# FASCICULE DE BREVET EUROPEEN

(11) **EP 3 145 577 B1**
(45) Date de publication et mention de la délivrance du brevet: **06.05.2020**
(21) Numéro de dépôt: 15728629.5
(22) Date de dépôt: 12.05.2015
(51) Int. Cl.: A61N 1/04

(54) **CONNECTEUR ELECTRIQUE NOTAMMENT POUR DISPOSITIF CUTANE**
ELEKTRISCHER VERBINDER, INSBESONDERE FÜR EINE KUTANE VORRICHTUNG
ELECTRICAL CONNECTOR, IN PARTICULAR FOR A CUTANEOUS DEVICE

(30) Priorité: 19.05.2014 FR 1454451
(43) Date de publication de la demande: 29.03.2017
(73) Titulaire: Commissariat à l'Énergie Atomique et aux Énergies Alternatives, 75015 Paris (FR)
(72) Inventeur: PERRAUD, Simon, F-83150 Bandol (FR); EMIEUX, Fabrice, F-38340 Voreppe (FR); KARST, Nicolas, F-57600 Folkling (FR); PANTIGNY, Philippe, F-38640 Claix (FR)
(74) Mandataire: Gevers & Orès
(86) Numéro de dépôt international: PCT/IB2015/053484
(87) Numéro de publication internationale: WO 2015/177682

(56) Documents cités:
- FR-A1- 2 991 589
- US-A- 4 025 964
- US-A- 4 479 685
- US-A1- 2004 176 675
- US-A1- 2007 026 695
- US-A1- 2009 306 741
- US-A1- 2013 023 816

## Description

L'invention concerne le domaine technique des dispositifs destinés à être fixés sur la peau d'un utilisateur.

Il s'agit notamment de dispositifs médicaux, comme des électrodes cutanées pour la mesure de paramètres physiologiques ou des générateurs d'impulsions pour l'électrostimulation.

Ces dispositifs sont en général suffisamment compacts pour être placés directement sur la peau d'un utilisateur. Ils sont conçus pour être également relativement fins et flexibles.

Ces dispositifs comprennent généralement un connecteur électrique permettant de connecter le dispositif à un fil électrique.

Le connecteur électrique est constitué de deux parties :
- une fiche, c'est-à-dire la partie du connecteur située à l'extrémité du fil électrique,
- une embase, c'est-à-dire la partie du connecteur solidaire du dispositif.

Un connecteur électrique couramment utilisé est du type *« bouton pression* ». Généralement, la fiche comprend une cavité (fiche femelle) et l'embase comprend une protubérance (embase mâle) destinée à s'insérer dans la cavité.

Les connecteurs électriques du type « *bouton pression »* présentent des inconvénients, notamment dans le cas où l'utilisateur fixe le dispositif sur sa peau avant d'effectuer la connexion.

En effet, une pression (c'est-à-dire une force perpendiculaire au plan du dispositif) doit être exercée pour effectuer la connexion, ce qui peut être douloureux pour l'utilisateur.

De plus, l'utilisateur doit positionner la cavité de la fiche exactement en face de la protubérance de l'embase avant d'exercer la pression. Ainsi, un contrôle visuel est nécessaire pour effectuer rapidement la connexion. De ce fait, effectuer la connexion peut devenir une opération extrêmement longue et fastidieuse dans le cas où ce contrôle visuel n'est pas possible. Cela se produit par exemple, dans le cas où l'utilisateur a fixé le dispositif dans son dos où derrière l'une de ses jambes.

Ainsi, avec un connecteur électrique du type « *bouton pression* », il est généralement préférable de fixer le dispositif sur la peau après avoir effectué la connexion électrique, ce qui est moins pratique pour l'utilisateur.

D'autres types de connexion ont été proposés dans l'art antérieur, comme US-2013/023816 et US-2007/026695.

En particulier, des connecteurs électriques comprenant des pièces magnétiques sont décrits dans le document US-4112941.

Ce document concerne spécifiquement le domaine des électrodes cutanées, avec une fiche comprenant une cavité (fiche femelle) et une embase comprenant une protubérance (embase mâle). Avec le connecteur électrique décrit dans ce document, il n'est plus nécessaire d'exercer de pression pour effectuer la connexion, car la force magnétique entre la fiche et l'embase suffit à insérer la protubérance de l'embase dans la cavité de la fiche.

Ceci résout donc l'un des problèmes rencontrés avec les connecteurs électriques du type « *bouton pression* », puisqu'il n'est plus nécessaire d'exercer une pression pour réaliser la connexion.

Cependant, un contrôle visuel est toujours nécessaire pour effectuer rapidement la connexion. En effet, l'utilisateur doit au moins pré-positionner la cavité de la fiche à proximité de la protubérance de l'embase, avant que les forces magnétiques n'entrent en action et n'assemblent les deux parties du connecteur.

L'invention a pour objet de pallier ces inconvénients en proposant un connecteur électrique, notamment pour un dispositif médical destiné à être fixé sur la peau d'un utilisateur, comprenant une embase solidaire du dispositif et une fiche destinée à être solidaire d'un conducteur électrique, qui permet d'effectuer une connexion rapide de l'embase et de la fiche sans contrôle visuel et, de préférence, sans exercer de pression.

Selon l'invention, la fiche comprend un moyen de connexion et l'embase comprend une pluralité de moyens de connexion, chacun d'eux étant adaptés pour coopérer avec le moyen de connexion de la fiche pour assurer la connexion entre l'embase et la fiche.

Ainsi, l'embase comporte une densité importante de zones de connexion potentielles avec la fiche, ce qui permet de réaliser une connexion rapide entre l'embase et la fiche et ceci, sans contrôle visuel.

De préférence, la distance centre à centre entre deux moyens de connexion de l'embase adjacents est comprise entre 1,5 et 10 fois la section de ces moyens de connexion.

Par ailleurs, les moyens de connexion de l'embase sont, de préférence, répartis de façon uniforme sur la surface de l'embase.

Le connecteur électrique selon l'invention comporte, de préférence, des moyens magnétiques à la fois sur la fiche et l'embase.

La présence de ces moyens magnétiques évite à l'usager d'exercer une pression pour effectuer la connexion entre la fiche et l'embase.

Ils contribuent donc à faciliter la connexion entre la fiche et l'embase, sans contrôle visuel.

Dans un premier mode de réalisation, l'embase comporte une pluralité de trous et la fiche comporte une protubérance.

Dans ce premier mode de réalisation du connecteur, l'embase est constituée par un empilement d'au moins deux couches, l'une d'elles remplissant au moins une fonction de support mécanique et lesdites au moins deux couches remplissant également une fonction électrique et éventuellement une fonction magnétique, les trous de l'embase traversant au moins la couche remplissant la fonction de support mécanique, et la fiche comprenant une base et une protubérance remplissant une fonction électrique et éventuellement une fonction magnétique.

Dans une première forme de réalisation, l'empilement constituant l'embase comprend une couche remplissant une fonction de support mécanique et une couche remplissant une fonction électrique et une éventuelle fonction magnétique, les trous traversant l'empilement.

En outre, la fonction électrique et éventuellement magnétique de la fiche est remplie par la base,

Dans une deuxième forme de réalisation du premier mode de réalisation du connecteur, l'empilement constituant l'embase comprend une couche remplissant une fonction électrique et une couche remplissant une fonction mécanique, la couche remplissant une fonction électrique pouvant remplir également une fonction magnétique, les trous traversant uniquement la couche remplissant une fonction mécanique, la fonction électrique et éventuellement magnétique de la fiche étant remplie par la protubérance.

Dans une troisième forme de réalisation du premier mode de réalisation du connecteur, l'empilement constituant l'embase comprend successivement une couche remplissant une fonction électrique, une couche remplissant une fonction mécanique et éventuellement une couche remplissant une fonction magnétique, les trous traversant la couche remplissant une fonction mécanique et, lorsqu'elle est présente, la couche remplissant une fonction magnétique, l'éventuelle fonction magnétique de la fiche étant remplie par la base et la fonction électrique de la fiche étant remplie par la protubérance.

Dans une première configuration particulière, l'éventuelle fonction magnétique est remplie par la couche remplissant une fonction mécanique.

Dans une deuxième configuration particulière, une couche ayant une fonction esthétique est prévue sur la couche remplissant une fonction mécanique ou sur l'éventuelle couche remplissant une fonction magnétique.

Dans une première variante de réalisation, la fiche comprend une pièce annulaire en saillie sur la base et s'étendant autour de la protubérance, l'embase comprenant autour de chaque trou, une ouverture annulaire pour recevoir la pièce annulaire en saillie de la fiche.

Dans une deuxième variante de réalisation, la fiche comporte une protubérance présentant une forme évasée se rétrécissant en s'éloignant de la base, les trous de l'embase comportant une forme correspondante pour recevoir cette protubérance.

Dans une troisième variante de réalisation, la protubérance de la fiche comprend deux pièces remplissant une fonction électrique et éventuellement une fonction magnétique, séparées par un isolant.

Dans une quatrième variante de réalisation, la protubérance de la fiche présente une forme évasée s'élargissant en s'éloignant de la base, les trous de l'embase présentant une forme correspondante pour recevoir cette protubérance.

Dans une cinquième variante, la couche de l'embase qui n'est pas traversée par les trous est une couche discontinue, se présentant notamment sous la forme d'une grille ou de pièces indépendantes et non connectées électriquement.

La cinquième variante de réalisation est applicable aux deuxième et troisième formes de réalisation du premier mode de réalisation du connecteur.

La sixième variante de réalisation est applicable à la première et à la troisième forme de réalisation du premier mode de réalisation.

Dans cette sixième variante, une couche traversée par les trous est une couche discontinue.

Dans une septième variante de réalisation, la protubérance de la fiche remplit une fonction élastique pour établir un meilleur contact électrique.

Dans une huitième variante de réalisation applicable à la deuxième et à la troisième forme de réalisation, la couche remplissant une fonction électrique est constituée de deux pièces distinctes et non connectées électriquement, la protubérance ou la base de la fiche permettant de connecter électriquement ces deux pièces lorsque la fiche est connectée à l'embase.

Dans un deuxième mode de réalisation, l'embase comporte une pluralité de protubérances et la fiche comporte une cavité.

Dans ce cas, l'embase comprend au moins une couche remplissant une fonction de support mécanique et la pluralité de protubérances est réalisée directement sur la couche de support mécanique ou sur une couche intermédiaire, les protubérances, la couche remplissant une fonction de support mécanique ou la couche intermédiaire remplissant une fonction électrique et éventuellement une fonction magnétique, et la fiche comprend une pièce cylindrique définissant la cavité qui est fermée par une base, la fonction électrique de la fiche étant remplie par la pièce cylindrique ou la base et l'éventuelle fonction magnétique de la fiche étant remplie par la pièce cylindrique ou la base.

Dans une première forme de réalisation de ce deuxième mode de réalisation du connecteur, l'embase comprend sur la couche remplissant une fonction de support mécanique, une éventuelle couche intermédiaire, la fonction électrique et l'éventuelle fonction magnétique étant remplie(s) par la couche remplissant une fonction de support mécanique ou l'éventuelle couche intermédiaire, les protubérances étant réalisées sur la couche remplissant une fonction de support mécanique ou sur la couche intermédiaire, la fonction électrique et l'éventuelle fonction magnétique de la fiche étant remplie(s) par la pièce cylindrique.

Dans une deuxième forme de réalisation, l'embase comprend, sur la couche remplissant une fonction de support mécanique, des protubérances réalisées directement sur cette couche de support mécanique, ces protubérances remplissant une fonction électrique et éventuellement une fonction magnétique, la fonction électrique et l'éventuelle fonction magnétique de la fiche étant remplie(s) par la base.

Dans une troisième forme de réalisation du deuxième mode de réalisation du connecteur, l'embase comprend, sur la couche remplissant une fonction de support mécanique, une couche intermédiaire remplissant une fonction électrique, les protubérances étant réalisées sur cette couche intermédiaire et remplissant éventuellement une fonction magnétique, la pièce cylindrique de la fiche remplissant une fonction électrique et sa base remplissant éventuellement une fonction magnétique.

Dans une variante de cette troisième forme de réalisation, ce sont les protubérances qui remplissent une fonction électrique et la couche intermédiaire qui remplit éventuellement une fonction magnétique.

Le connecteur selon l'invention peut comporter une pluralité de fiches, dont le nombre est strictement inférieur à celui des moyens de connexion de l'embase.

L'invention concerne également un dispositif médical destiné à être fixé sur la peau d'un utilisateur, du type patch.

Dans ce cas, les moyens de connexion de l'embase sont avantageusement répartis de façon à occuper plus de 1% de la surface totale du dispositif et notamment, plus de 5% de cette surface.

L'invention sera mieux comprise et d'autres buts, avantages et caractéristiques de celle-ci apparaîtront plus clairement à la lecture de la description qui suit et qui est faite au regard des dessins annexés, sur lesquels :
- la figure 1 (1A-1B) comprend une vue de dessus (figure 1A) et une vue en coupe (figure 1B) d'une embase femelle correspondant à un premier mode de réalisation du connecteur,
- la figure 2 (2A-2F) représente vus de dessus (figures 2A, 2C, 2E) et en coupe (figures 2B, 2D, 2F), trois exemples de fiches mâles adaptées à l'embase illustrée à la figure 1, l'embase selon la figure 1 étant illustrée en pointillés sur les figures 2B à 2F,
- la figure 3 (3A-3B) présente une vue de dessus (figure 3A) et une vue en coupe (figure 3B) d'une embase femelle correspondant à une deuxième forme de réalisation du connecteur selon l'invention,
- la figure 4 (4A-4F) présente vus de dessus (figures 4A, 4C, 4E) et vus en coupe (figures 4B, 4D, 4F), des exemples de fiches mâles adaptées à l'embase illustrée à la figure 3, cette embase étant partiellement illustrée en pointillés sur les figures 4B, 4D et 4F,
- la figure 5 (5A-5B) comprend une vue de dessus (figure 5A) et une vue en coupe (figure 5B) d'une embase femelle correspondant à une troisième forme de réalisation du connecteur, et adaptée à la fiche mâle illustrée à la figure 6,
- la figure 6 (6A-6B) comprend une vue de dessus (figure 6A) et une vue en coupe (figure 6B) d'un exemple d'une fiche mâle adaptée à l'embase illustrée à la figure 5,
- la figure 7 (7A-7B) comprend une vue de dessus (figure 7A) et une vue en coupe (figure 7B) d'une première variante de réalisation d'une fiche mâle adaptée aux embases illustrées aux figures 1, 3 et 5,
- la figure 8 (8A-8B) comprend une vue de dessus (figure 8A) et une vue en coupe (figure 8B) d'une embase femelle du type illustré à la figure 1 et adaptée à la variante de la fiche illustrée à la figure 7, la fiche illustrée à la figure 7 étant montrée connectée à l'embase sur la figure 8B,
- la figure 9 est une vue en coupe d'une deuxième variante de réalisation d'une fiche mâle, destinée à être connectée à une embase femelle du type illustré aux figures 1, 3 et 5,
- la figure 10 est une vue en coupe d'une embase femelle du type illustré à la figure 1, adaptée à la fiche illustrée à la figure 9, cette fiche étant également illustrée connectée à cette embase,
- la figure 11 est une vue en coupe illustrant une troisième variante de réalisation d'une fiche mâle adaptée à l'une des embases femelles illustrées à l'une des figures 3 ou 5,
- la figure 12 (12A-12B) comprend une vue de dessus (figure 12A) et une vue en coupe (figure 12B) d'une embase du type illustré à la figure 3 et adapté à la fiche illustrée à la figure 11,
- la figure 13 est une vue en coupe représentant une quatrième variante de réalisation d'une fiche mâle adaptée aux embases femelles décrites en référence aux figures 1, 3 et 5,
- la figure 14 est une vue de dessus d'une embase du type illustré à la figure 1 et adaptée à la fiche illustrée à la figure 3,
- la figure 15 (15A-15E) présente des vues de dessus d'exemples de la couche inférieure d'une embase femelle, telle qu'illustrée aux figures 3 et 5, correspondant à une cinquième variante de réalisation,
- la figure 16 est une vue en coupe d'une embase du type illustré à la figure 15D selon la cinquième variante de réalisation, une fiche mâle du type illustré à la figure 6 étant connectée sur cette embase,
- la figure 17 (17A-17B) comprend une vue de dessus (figure 17A) et une vue en coupe (figure 17B) représentant une sixième variante de réalisation de l'embase illustrée à la figure 5,
- la figure 18 est une vue en coupe d'une électrode cutanée comprenant une embase selon la figure 5,
- la figure 19 (19A-19B) comprend une vue en coupe (figure 19A) d'un patch générant des impulsions et incluant une embase selon la figure 15D et une vue de dessus (figure 19B) du schéma électrique du patch illustré à la figure 19A,
- la figure 20 (20A-20B) comprend la figure 20A montrant les dispositifs illustrés aux figures 18 et 19 en place sur le corps d'un utilisateur, l'électrode selon la figure 18 et du patch selon la figure 19 sur le corps d'un utilisateur et la figure 20B illustrant la bande élastique connectant ces deux dispositifs,
- la figure 21 (21A-21B) illustre une première forme de réalisation d'une embase mâle représentée vue de dessus (figure 21A) et vue en coupe (figure 21B),
- la figure 22 (22A-22B) représente vue de dessus (22A) et vue en coupe (22B), une fiche femelle adaptée à l'embase illustrée à la figure 21,
- la figure 23 (23A-23B) illustre une deuxième forme de réalisation d'une embase mâle, la figure 23A illustrant cette embase vue de dessus et la figure 23B l'illustrant vue en coupe,
- la figure 24 (24A-24B) illustre vue de dessus (figure 24A) et vue en coupe (figure 24B) une fiche femelle adaptée à l'embase illustrée à la figure 23,
- la figure 25 (25A-25B) illustre une troisième forme de réalisation d'une embase mâle, vue de dessus (figure 25A) et vue en coupe (figure 25B),
- la figure 26 (26A-26B) représente vue de dessus (figure 26A) et vue en coupe (figure 26B) une fiche femelle adaptée à l'embase mâle illustrée à la figure 25.

Les éléments communs aux différentes figures seront désignés par les mêmes références.

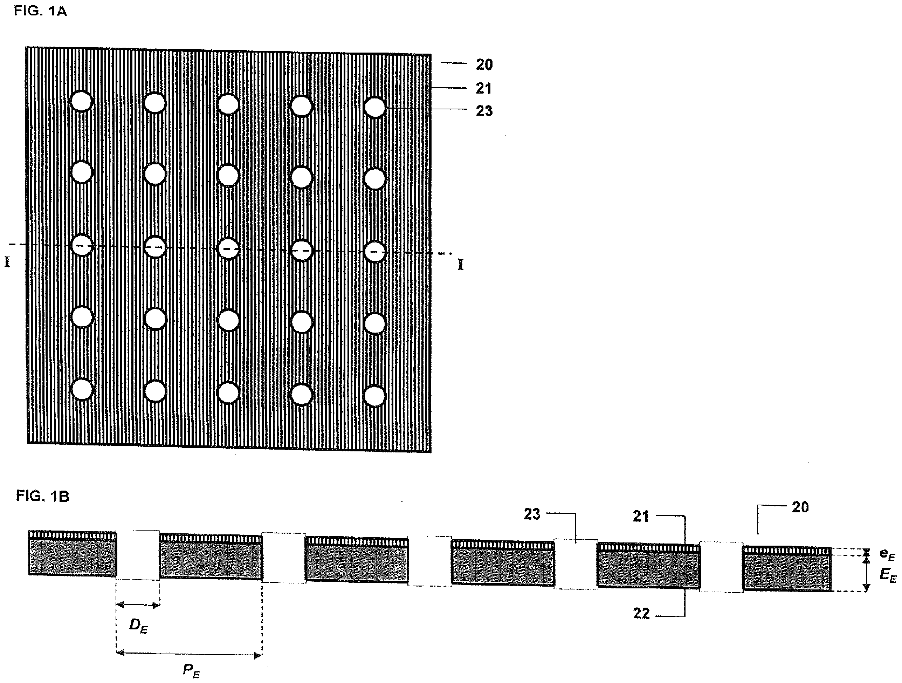

Les figures 1 à 6 illustrent plusieurs formes de réalisation d'un connecteur selon l'invention comportant une embase femelle avec une pluralité de trous et une fiche mâle avec une protubérance.

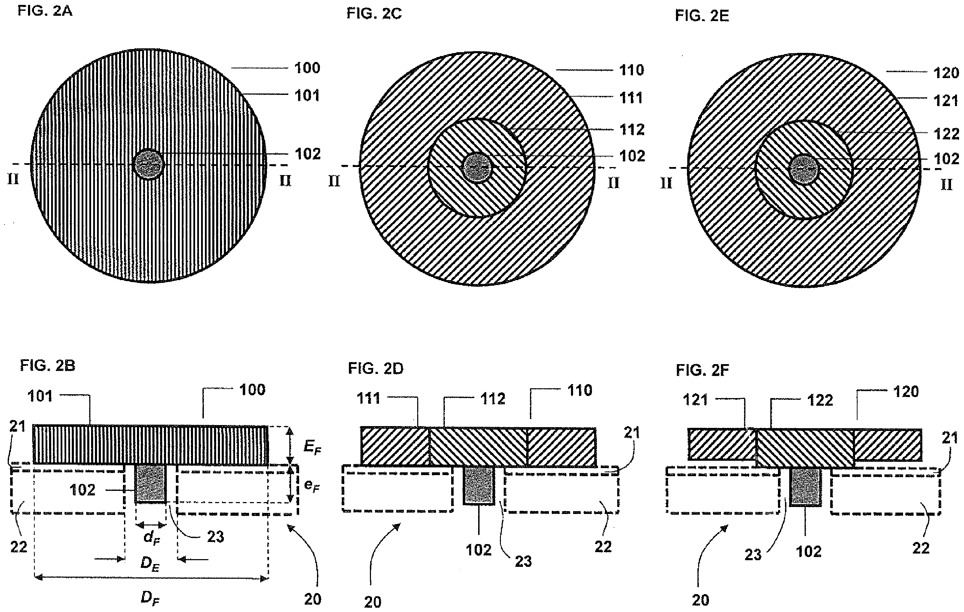

Ainsi, les figures 1 et 2 illustrent une première forme de réalisation d'un tel connecteur dans laquelle l'embase comprend :
- une couche supérieure ayant une fonction électrique et magnétique,
- une couche inférieure ayant une fonction mécanique,
- une pluralité de trous traversant la couche supérieure et la couche inférieure.

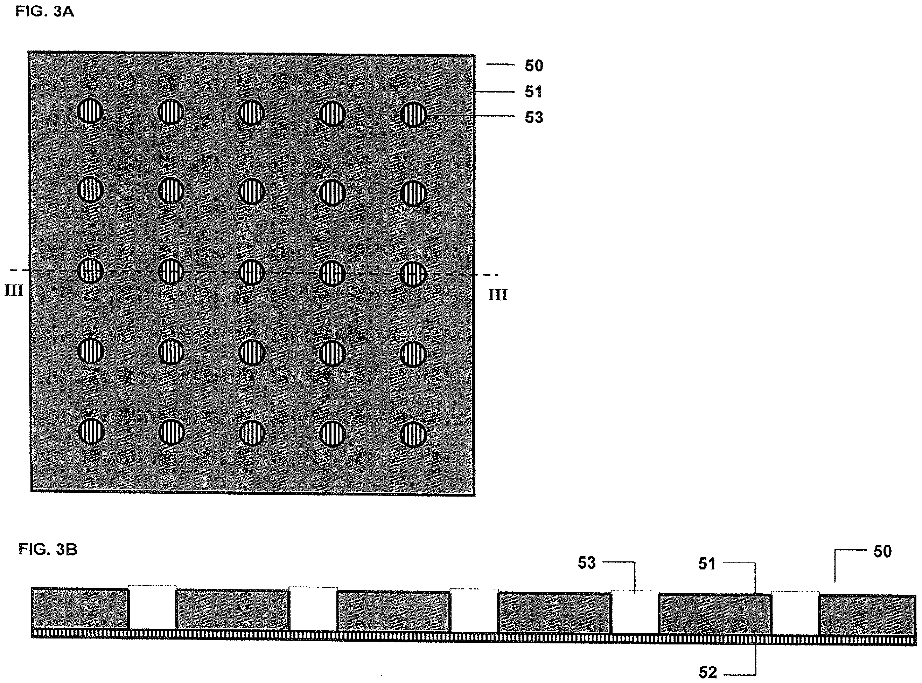

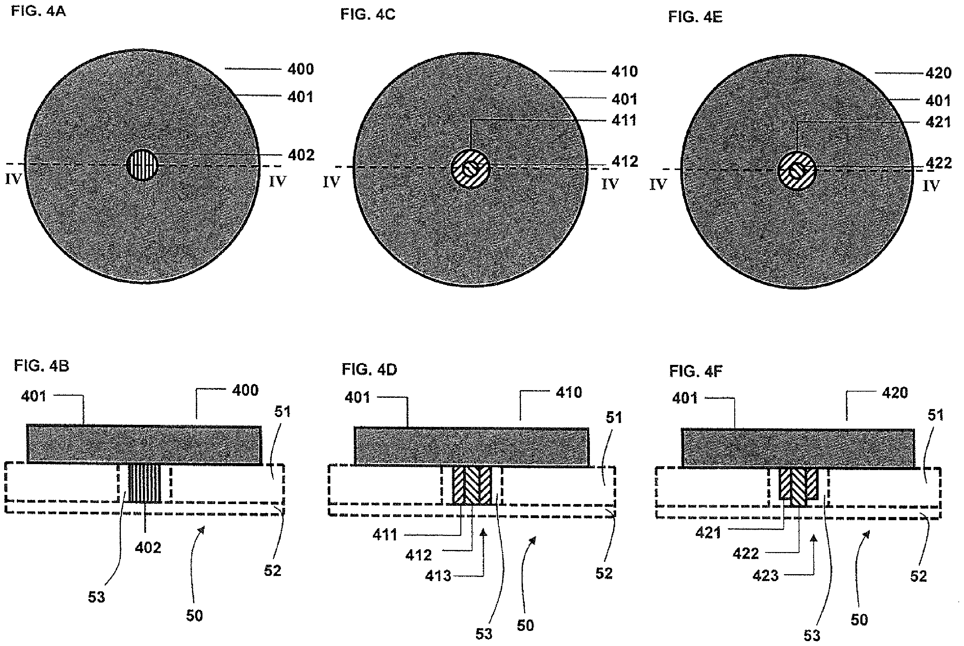

Les figures 3 et 4 illustrent une deuxième forme de réalisation d'un tel connecteur dans laquelle l'embase comprend :
- une couche supérieure ayant une fonction mécanique,
- une couche inférieure ayant une fonction électrique et magnétique,
- une pluralité de trous traversant seulement la couche supérieure.

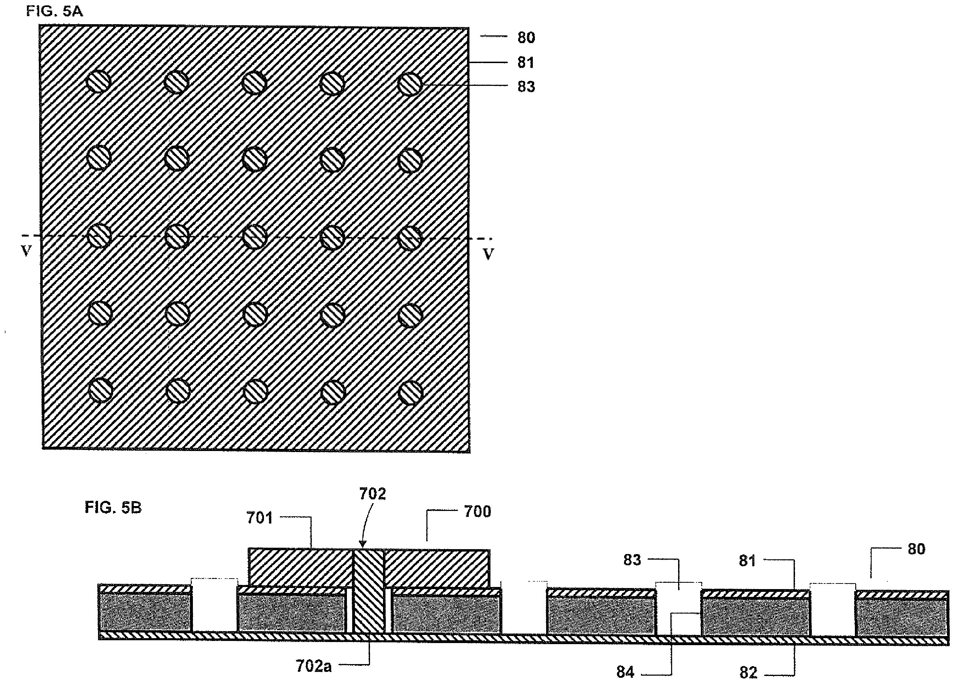

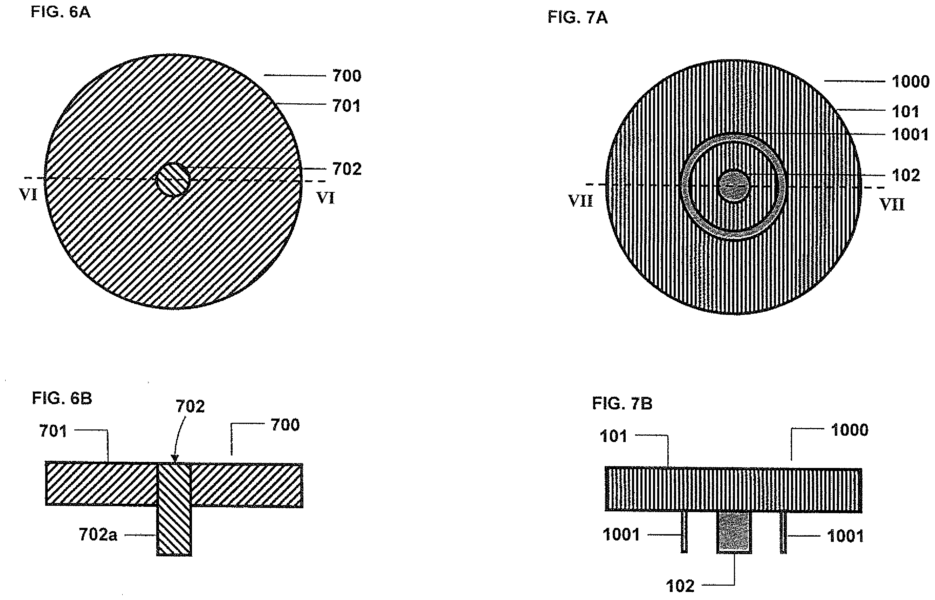

Les figures 5 et 6 illustrent une troisième forme de réalisation d'un tel connecteur dans laquelle l'embase comprend :
- une couche supérieure ayant une fonction magnétique,
- une couche intermédiaire ayant une fonction mécanique,
- une couche inférieure ayant une fonction électrique,
- une pluralité de trous traversant seulement la couche supérieure et la couche intermédiaire.

Une première configuration de cette troisième forme de réalisation (non illustrée sur les figures) consiste à supprimer la couche supérieure, l'éventuelle fonction magnétique étant remplie par la couche intermédiaire.

Une deuxième configuration de cette troisième forme de réalisation (non illustrée sur les figures) consiste à ajouter une couche, ayant notamment une fonction esthétique, au-dessus de la couche ayant une fonction magnétique. Ainsi, l'embase comprend alors quatre couches :
- une couche ayant une fonction esthétique,
- une couche ayant une fonction magnétique,
- une couche ayant une fonction mécanique,
- une couche ayant une fonction électrique,
- une pluralité de trous traversant seulement la couche ayant une fonction esthétique, la couche ayant une fonction magnétique et la couche ayant une fonction mécanique.

Lorsque la couche ayant une fonction mécanique a également une fonction magnétique, la couche ayant une fonction esthétique est directement prévue sur cette couche.

Dans chacune de ces trois formes de réalisation :
- La couche de l'embase ayant une fonction électrique est destinée à établir un contact électrique avec la pièce de la fiche ayant une fonction électrique.
La couche de l'embase ayant une fonction électrique est formée dans un matériau conducteur de l'électricité et par exemple un métal, tel que le cuivre, l'argent, l'or, l'aluminium, le fer, ou l'acier ; ou un polymère chargé de noir de carbone ou d'éléments métalliques ; ou encore un matériau textile conducteur.
Avantageusement, il s'agit d'une couche fine, voire flexible.
Dans le cas où la couche est formée dans un métal ou un alliage de métaux, l'épaisseur de la couche est avantageusement comprise entre 0.001 mm et 1 mm et, de préférence, entre 0.01 mm et 0.2 mm. Dans le cas où la couche est formée dans un polymère chargé de noir de carbone ou de d'éléments métalliques, ou dans un matériau textile conducteur, l'épaisseur de la couche est avantageusement comprise entre 0.1 mm et 5 mm et, de préférence, entre 0.5 mm et 2 mm.
- La couche de l'embase ayant une fonction magnétique et la pièce de la fiche ayant une fonction magnétique sont destinées à exercer l'une sur l'autre une force magnétique attractive.
La couche de l'embase ayant une fonction magnétique est formée dans un matériau ferromagnétique, par exemple fer, acier magnétique, cobalt, nickel, ou polymère chargé d'éléments ferromagnétiques ou dans un matériau aimanté, par exemple aluminium-nickel-cobalt, samarium-cobalt, néodyme-fer-bore, ou polymère chargé d'éléments aimantés.
Avantageusement, il s'agit d'une couche fine, voire flexible.
Dans le cas où la couche de l'embase est formée en un métal ou un alliage de métaux, l'épaisseur de la couche est avantageusement comprise entre 0.001 mm et 1 mm et, de préférence, entre 0.01 mm et 0.2 mm. Dans le cas où la couche est formée en un polymère chargé d'éléments ferromagnétiques ou aimantés, l'épaisseur de la couche est avantageusement comprise entre 0.1 mm et 5 mm et, de préférence, entre 0.5 mm et 2 mm.
- La couche de l'embase ayant une fonction mécanique est traversée par les trous.
L'un quelconque de ces trous est destiné à accueillir la protubérance de la fiche et à empêcher ainsi une déconnexion accidentelle due à des forces latérales, c'est-à-dire des forces dans le plan de l'embase.
La couche de l'embase ayant une fonction mécanique est suffisamment épaisse pour assurer le maintien latéral de la protubérance et donc de la fiche.
Avantageusement, l'épaisseur de la couche est comprise entre 0.1 mm et 5 mm et, de préférence, entre 0.5 mm et 2 mm.
Avantageusement, la couche est formée dans un matériau très souple (par exemple un polymère ou un matériau textile), afin de rester flexible malgré son épaisseur relativement importante.
- La densité surfacique de trous sur l'embase est suffisamment élevée pour permettre une connexion rapide sans contrôle visuel.
Ceci est facilité par l'action des forces magnétiques. Cependant, cette densité de trous permet d'obtenir une connexion sans contrôle visuel, même en l'absence de pièces remplissant une fonction magnétique à l'intérieur du connecteur.
Cependant, en présence de pièces remplissant une fonction magnétique, on constate qu'un petit déplacement latéral de la fiche à proximité de la surface de l'embase suffit pour que les forces magnétiques entrent en action et assemblent les deux parties du connecteur, en provoquant l'insertion de la protubérance de la fiche dans l'un des trous de l'embase.
Autrement dit, la forte densité surfacique de zones de connexion potentielles (les trous de l'embase), combinée à l'action des forces magnétiques, rend encore plus rapide et simple une connexion sans contrôle visuel.
Avantageusement, le diamètre des trous (en considérant des trous cylindriques) est compris entre 0.1 mm et 10 mm et, de préférence, entre 0.5 mm et 5 mm. Avantageusement, la distance centre à centre entre deux trous voisins de diamètre identique est comprise entre 1.5 fois le diamètre d'un trou et 10 fois le diamètre et, de préférence, entre 1.5 fois le diamètre et 5 fois le diamètre. Les trous n'ont pas nécessairement une forme circulaire, mais peuvent être par exemple de forme oblongue.
Avantageusement, les trous de l'embase sont répartis de façon uniforme sur la surface de l'embase.
Dans le cas où l'embase est destinée à un dispositif porté sous la forme d'un patch, la somme des surfaces occupées par les trous correspond avantageusement à plus de 1% de la surface totale du patch et, de préférence, à plus de 5% de la surface totale du patch.

Par ailleurs, dans toutes ces formes de réalisation, la fiche comprend :
- une pièce ayant une fonction de protubérance,
- une pièce ayant une fonction électrique (c'est-à-dire une pièce formée dans un matériau conducteur de l'électricité, par exemple un métal, tel que le cuivre, l'argent, l'or, l'aluminium, le fer, ou l'acier ; ou un polymère chargé de noir de carbone ou d'éléments métalliques),
- une pièce ayant une fonction magnétique (c'est-à-dire une pièce formée dans un matériau ferromagnétique, par exemple fer, acier magnétique, cobalt, nickel ; ou une pièce formée dans un matériau aimanté, par exemple ferrite, aluminium-nickel-cobalt, samarium-cobalt, néodyme-fer-bore).

En référence tout d'abord aux figures 1 et 2, les figures 1A (vue de dessus) et 1B (vue en coupe selon la ligne I-I de la figure 1A) représentent l'embase 20 du connecteur électrique.

L'embase 20 comprend une couche supérieure 21 (d'épaisseur *e_{E}*) ayant une fonction électrique et magnétique, et une couche inférieure 22 (d'épaisseur *E_{E}*) ayant une fonction mécanique.

L'embase 20 comprend une pluralité de trous cylindriques 23 (de diamètre *D_{E}*) traversant la couche supérieure 21 et la couche inférieure 22.

Les trous 23 forment ici un réseau périodique (de période *P_{E}*). La couche supérieure 21 est par exemple une feuille en acier inoxydable magnétique ; il peut s'agir d'une feuille flexible.

La couche inférieure 22 est par exemple une couche en polymère ou en matériau textile ; il peut s'agir d'une couche flexible.

Avantageusement, l'épaisseur *e_{E}* de la couche supérieure 21 est comprise entre 0.001 mm et 0.5 mm et, de préférence, entre 0.01 mm et 0.2 mm. Par exemple, la couche supérieure 21 est une feuille flexible en acier inoxydable magnétique AISI 420, d'épaisseur *e_{E}* = 0.075 mm.

Avantageusement, l'épaisseur *E_{E}* de la couche inférieure 22 est comprise entre 0.1 mm et 5 mm et, de préférence, entre 0.5 mm et 2 mm. Par exemple, la couche inférieure 22 est une couche flexible, en élastomère de silicone ou en mousse de polyuréthane, d'épaisseur *E_{E}* = 1 mm.

Le diamètre *D_{E}* des trous 23 est avantageusement compris entre 0.1 mm et 10 mm et, de préférence, entre 0.5 mm et 5 mm. La période *P_{E}* du réseau de trous 23 est avantageusement comprise entre 1.5 × *D_{E}* et 10 × *D_{E}* et, de préférence, entre 1.5 × *D_{E}* et 5 × *D_{E}.*

Les figures 2A (vue de dessus) et 2B (vue en coupe selon la ligne II-II sur la figure 2A) représentent un premier exemple de fiche 100 du connecteur électrique.

Cette fiche est solidaire d'un conducteur électrique, par exemple un fil ou un câble électrique (non représenté sur les figures).

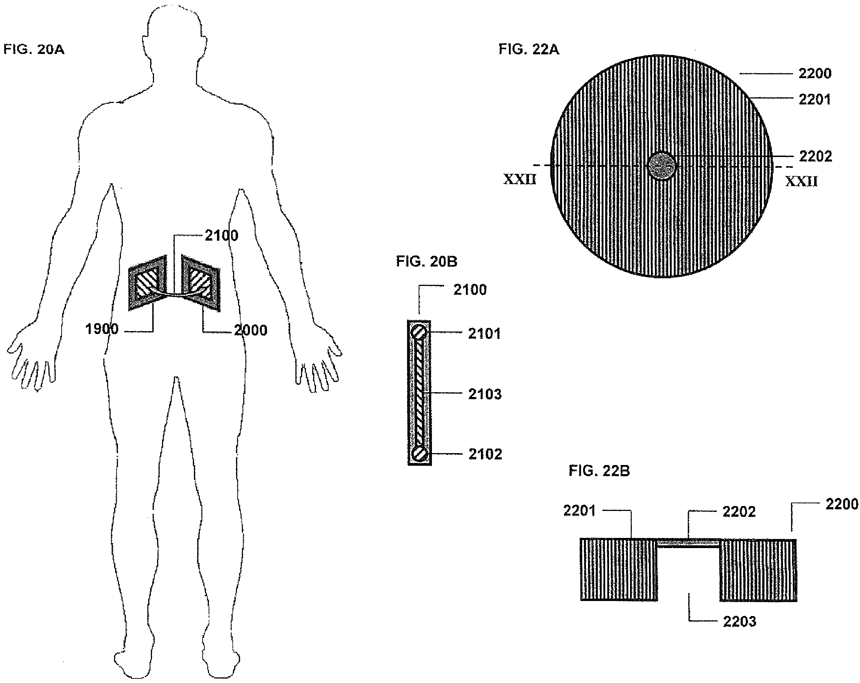

Des exemples de tels conducteurs électriques seront décrits en référence à la figure 20.

La fiche 100 comprend une pièce cylindrique 101, de diamètre *D_{F}* et d'épaisseur *E_{F}*, ayant une fonction électrique et magnétique, et une pièce cylindrique 102, de diamètre *d_{F}* et d'épaisseur *e_{F}*, formant une protubérance.

Les deux pièces cylindriques 101 et 102 sont centrées autour d'un même axe.

La pièce 101 est par exemple une pièce en aluminium-nickel-cobalt, ou samarium-cobalt, ou néodyme-fer-bore, qui sont des matériaux aimantés et conducteurs de l'électricité.

La protubérance 102 est par exemple une pièce en métal.

Les figures 2C (vue de dessus) et 2D (vue en coupe selon la ligne II-II sur la figure 2C) représentent un deuxième exemple de fiche 110.

A la place de la pièce 101, la fiche 110 comprend une pièce annulaire 111 ayant une fonction magnétique et une pièce cylindrique 112 ayant une fonction électrique, placée dans l'évidement central de la pièce 111. Les trois pièces 111, 112 et 102 sont centrées autour d'un même axe.

La pièce 111 est par exemple une pièce en ferrite, ou aluminium-nickel-cobalt, ou samarium-cobalt, ou néodyme-fer-bore, qui sont des matériaux aimantés. La pièce 112 est par exemple une pièce en métal.

Les figures 2E (vue de dessus) et 2F (vue en coupe selon la ligne II-II sur la figure 2E) représentent une fiche 120 qui est une variante de la fiche 110.

Dans la fiche 120, la pièce annulaire 121 ayant une fonction magnétique est légèrement en retrait (avantageusement de quelques millimètres) de la pièce cylindrique 122 ayant une fonction électrique.

Le diamètre extérieur *D_{F}* de la pièce 101, 111 ou 121 est supérieur à *D_{E}.* Le diamètre *d_{F}* de la protubérance 102 est inférieur à *D_{E}.*

L'épaisseur *e_{F}* de la protubérance 102 est avantageusement légèrement inférieure à la somme *e_{E}* + *E_{E}.* Par exemple, *e_{E}* = 0.075 mm, *E_{E}* = 1 mm, *D_{E}* = 1 mm, *P_{E}* = 4 mm, *D_{F}* = 6 mm, *E_{F}* = 1 mm, *d_{F}* = 0.8 mm, et *e_{F}* = 0.8 mm.

La figure 2B représente également la fiche 100 une fois connectée à l'embase 20 qui est partiellement représentée en pointillés.

L'un des trous 23 accueille la protubérance 102, ce qui empêche une déconnexion accidentelle due à des forces latérales, c'est-à-dire des forces dans le plan de l'embase 20. La couche supérieure 21 établit un contact électrique avec la pièce 101. La couche supérieure 21 et la pièce 101 exercent l'une sur l'autre une force magnétique attractive.

L'intensité de la force magnétique entre la couche supérieure 21 et la pièce 101 dépend notamment de la nature et des dimensions de la couche supérieure 21 et de la pièce 101.

D'après les lois physiques du magnétisme, l'intensité de la force magnétique dépend également de l'architecture de la fiche 100 contenant la pièce 101. Par exemple, en plaçant la pièce aimantée 101 dans une pièce ferromagnétique en forme de coupelle (non représenté sur la figure 2B), il est possible de concentrer le flux magnétique sur les bords libres de la pièce aimantée 101, ce qui augmente la force magnétique. Avantageusement, l'intensité de la force magnétique est comprise entre 100 g et 1000 g. Une telle gamme d'intensité est atteignable avec les matériaux et les dimensions mentionnés précédemment, notamment si la pièce 101 est en néodyme-fer-bore.

La figure 2D représente également la fiche 110 une fois connectée à l'embase 20.

La couche supérieure 21 établit un contact électrique avec la pièce 112. La couche supérieure 21 et la pièce 111 exercent l'une sur l'autre une force magnétique attractive.

La figure 2F représente également la fiche 120 une fois connectée à l'embase 20.

La couche supérieure 21 établit un contact électrique avec la pièce 122. La couche supérieure 21 et la pièce 121 exercent l'une sur l'autre une force magnétique attractive.

Etant donné que la pièce 121 est légèrement en retrait de la pièce 122, la pression exercée par la pièce 122 sur la couche supérieure 21 est augmentée, et donc le contact électrique entre la couche supérieure 21 et la pièce 122 est amélioré. En d'autres termes, la résistance électrique du contact est diminuée.

En référence maintenant aux figures 3 et 4, les figures 3A (vue de dessus) et 3B (vue en coupe selon la ligne III-III sur la figure 3A) représentent l'embase 50 du connecteur électrique.

L'embase 50 comprend une couche supérieure 51 ayant une fonction mécanique, et une couche inférieure 52 ayant une fonction électrique et magnétique.

L'embase 50 comprend une pluralité de trous cylindriques 53 traversant seulement la couche supérieure 51. Les trous 53 forment ici un réseau périodique.

La couche supérieure 51 est par exemple une couche flexible en polymère ou en matériau textile ou encore en élastomère de silicone ou en mousse de polyuréthane, dont l'épaisseur est de 1 mm.

La couche inférieure 52 est par exemple une feuille flexible en acier inoxydable magnétique, par exemple en acier inoxydable magnétique AISI 420, dont l'épaisseur est de 0.075 mm.

Par rapport à la première forme de réalisation illustrée à la figure 1, un avantage de cette deuxième forme de réalisation réside dans le fait que la couche de l'embase ayant une fonction électrique n'est pas la couche supérieure de l'embase. Ceci apporte un gain du point de vue de la sécurité et de la fiabilité du dispositif.

En effet, l'utilisateur ne peut pas accéder facilement aux pièces destinées à être mises sous tension (telle que la couche ayant une fonction électrique), ce qui limite les risques électriques (pour l'utilisateur) et les risques de dysfonctionnement (pour le dispositif).

Les figures 4A (vue de dessus) et 4B (vue en coupe selon la ligne IV-IV sur la figure 4A) représentent un premier exemple de la fiche 400 du connecteur électrique.

La fiche 400 comprend une pièce cylindrique 401. La fiche 400 comprend une autre pièce cylindrique 402 formant une protubérance et remplissant une fonction électrique et magnétique.

Les deux pièces cylindriques 401 et 402 sont centrées autour d'un même axe.

La pièce 401 est par exemple une pièce en métal.

La protubérance 402 est par exemple une pièce en aluminium-nickel-cobalt, ou samarium-cobalt, ou néodyme-fer-bore, qui sont des matériaux aimantés et conducteurs de l'électricité.

Les figures 4C (vue de dessus) et 4D (vue en coupe selon la ligne IV-IV sur la figure 4C) représentent un deuxième exemple d'une fiche 410.

A la place de la protubérance 402, la fiche 410 comprend une protubérance 413 formée d'une pièce annulaire 411 ayant une fonction magnétique et d'une pièce cylindrique centrale 412 ayant une fonction électrique.

Les trois pièces 401, 411 et 412 sont centrées autour d'un même axe.

La pièce 411 est par exemple une pièce en ferrite, ou aluminium-nickel-cobalt, ou samarium-cobalt, ou néodyme-fer-bore, qui sont des matériaux aimantés. La pièce 412 est par exemple une pièce en métal.

Les figures 4E (vue de dessus) et 4F (vue en coupe selon la ligne IV-IV sur la figure 4E) représentent une fiche 420 qui est une variante de la fiche 410.

Dans la protubérance 423 de la fiche 420, la pièce annulaire 421 ayant une fonction magnétique est légèrement en retrait (avantageusement de quelques millimètres) de la pièce cylindrique centrale 422 ayant une fonction électrique.

La figure 4B représente également la fiche 400 une fois connectée à l'embase 50.

L'un des trous 53 accueille la protubérance 402, ce qui empêche une déconnexion accidentelle due à des forces latérales, c'est-à-dire des forces dans le plan de l'embase 50.

La couche inférieure 52 établit un contact électrique avec la protubérance 402.

La couche inférieure 52 et la protubérance 402 exercent l'une sur l'autre une force magnétique attractive.

Avantageusement, la hauteur de la protubérance 402 est égale ou légèrement supérieure à l'épaisseur de la couche 51.

Cependant, la hauteur de la protubérance 402 peut être légèrement inférieure à l'épaisseur de la couche 51. Dans ce cas, la compressibilité de la couche 51 ou la flexibilité de la couche 52 permettent de s'adapter à cette situation pour réaliser un contact électrique.

Afin d'améliorer le contact électrique entre la couche inférieure 52 et la protubérance 402, et d'éviter un encrassement de ces pièces pouvant conduire à un mauvais contact électrique, la couche inférieure 52 et la protubérance 402 peuvent avoir des formes différentes de celles représentées à la figure 4B.

En particulier, l'extrémité de la protubérance 402, c'est-à-dire la partie venant en contact avec la couche inférieure 52, n'est pas nécessairement plane, mais peut avoir une forme arrondie ou pointue. Dans ce cas, les zones exposées de la couche inférieure 52, c'est-à-dire les zones sur lesquelles débouchent les trous 53, ne sont pas nécessairement planes, mais peuvent être en forme de cavité permettant d'accueillir l'extrémité arrondie ou pointue de la protubérance 402.

Une autre méthode pour améliorer le contact électrique entre la protubérance 402 et l'embase 50 consiste à métalliser les parois internes des trous 53. Dans ce cas, la protubérance 402 peut établir un contact électrique via son extrémité ou via ses flancs.

La figure 4D représente également la fiche 410 une fois connectée à l'embase 50.

La couche inférieure 52 établit un contact électrique avec la pièce 412.

La couche inférieure 52 et la pièce 411 exercent l'une sur l'autre une force magnétique attractive.

La figure 4F représente également la fiche 420 une fois connectée à l'embase 50.

La couche inférieure 52 établit un contact électrique avec la pièce 422.

La couche inférieure 52 et la pièce 421 exercent l'une sur l'autre une force magnétique attractive.

Etant donné que la pièce 421 est légèrement en retrait de la pièce 422, la pression exercée par la pièce 422 sur la couche inférieure 52 est augmentée, et donc le contact électrique entre la couche inférieure 52 et la pièce 422 est amélioré. En d'autres termes, la résistance électrique du contact est diminuée.

En référence aux figures 5 et 6, les figures 5A (vue de dessus) et 5B (vue en coupe selon la ligne V-V sur la figure 5A) représentent l'embase 80 du connecteur électrique.

L'embase 80 comprend une couche supérieure 81 ayant une fonction magnétique, une couche intermédiaire 84 ayant une fonction mécanique, et une couche inférieure 82 ayant une fonction électrique.

L'embase 80 comprend une pluralité de trous cylindriques 83 traversant seulement la couche supérieure 81 et la couche intermédiaire 84. Les trous 83 forment ici un réseau périodique.

La couche supérieure 81 est par exemple une feuille flexible en acier inoxydable magnétique, notamment un acier inoxydable magnétique AISI 420, d'épaisseur 0.075 mm.

La couche intermédiaire 84 est par exemple une couche flexible en polymère ou en matériau textile, ou encore en élastomère de silicone ou en mousse de polyuréthane, dont l'épaisseur est de 1 mm.

La couche inférieure 82 est par exemple une bicouche métal/polymère (la couche en métal étant au-dessus de la couche en polymère) ou une bicouche cuivre/polyimide (avec une couche en cuivre d'épaisseur 0.035 mm et une couche en polyimide d'épaisseur 0.045 mm).

Par rapport à la première forme de réalisation illustrée à la figure 1, un avantage de cette troisième forme de réalisation réside dans le fait que la couche de l'embase ayant une fonction électrique n'est pas la couche supérieure de l'embase, ce qui apporte un gain du point de vue de la sécurité et de la fiabilité du dispositif.

Les figures 6A (vue de dessus) et 6B (vue en coupe selon la ligne VI-VI sur la figure 6A) représentent la fiche 700 du connecteur électrique.

La fiche 700 comprend une pièce annulaire 701 formant sa base, ayant une fonction magnétique, et une pièce cylindrique centrale 702 formant une protubérance 702a, en saillie par rapport à la pièce annulaire 701, et ayant une fonction électrique.

Les deux pièces 701 et 702 sont centrées autour d'un même axe.

La pièce 701 est par exemple une pièce en ferrite, ou aluminium-nickel-cobalt ou samarium-cobalt ou encore néodyme-fer-bore, qui sont des matériaux aimantés.

La protubérance 702 est par exemple une pièce en métal.

La figure 5B représente également la fiche 700 une fois connectée à l'embase 80.

L'un des trous 83 accueille la protubérance 702a, ce qui empêche une déconnexion accidentelle due à des forces latérales, c'est-à-dire des forces dans le plan de l'embase 80.

La couche inférieure 82 établit un contact électrique avec la protubérance 702. La couche supérieure 81 et la pièce 701 exercent l'une sur l'autre une force magnétique attractive.

La hauteur de la protubérance 702a est égale ou légèrement supérieure à la somme des épaisseurs des couches 81 et 84.

Afin d'améliorer le contact électrique entre la couche inférieure 82 et la protubérance 702a, et d'éviter un encrassement de ces pièces pouvant conduire à un mauvais contact électrique, la couche inférieure 82 et la protubérance 702a, peuvent avoir des formes différentes de celles représentées aux figures 5 et 6.

En particulier, l'extrémité de la protubérance 702a, c'est-à-dire la partie venant en contact avec la couche inférieure 82, n'est pas nécessairement plane, mais peut avoir une forme arrondie ou pointue.

Dans ce cas, les zones exposées de la couche inférieure 82, c'est-à-dire les zones sur lesquelles débouchent les trous 83, ne sont pas nécessairement planes, mais peuvent être en forme de cavité permettant d'accueillir l'extrémité arrondie ou pointue de la protubérance 702.

Une autre méthode pour améliorer le contact électrique entre la protubérance 702 et l'embase 80 consiste à métalliser les parois internes des trous 83. Dans ce cas, la protubérance 702 peut établir un contact électrique via son extrémité ou via ses flancs.

Dans toutes les formes de réalisation illustrées sur les figures 1 à 6, les trous de l'embase sont cylindriques, et la fiche possède une symétrie de révolution autour d'un axe perpendiculaire au plan de l'embase.

Ceci est avantageux, car ainsi la connexion électrique entre la fiche et l'embase peut être établie pour n'importe quelle orientation de la fiche. Il convient bien sûr que le plan de la fiche soit parallèle au plan de l'embase, ce qui est notamment assuré par les forces magnétiques.

Cependant, l'invention n'est pas limitée à cette forme de réalisation.

Par ailleurs, dans toutes les formes de réalisation illustrées sur les figures 1 à 6, l'embase et la fiche comportent une pièce remplissant une fonction magnétique.

Cependant, même en l'absence de pièces remplissant une fonction magnétique, les connecteurs décrits en référence à ces figures permettent de réaliser une connexion électrique sans contrôle visuel.

Les figures 7 à 17 représentent des variantes de réalisation du connecteur illustré sur les figures 1 à 6.

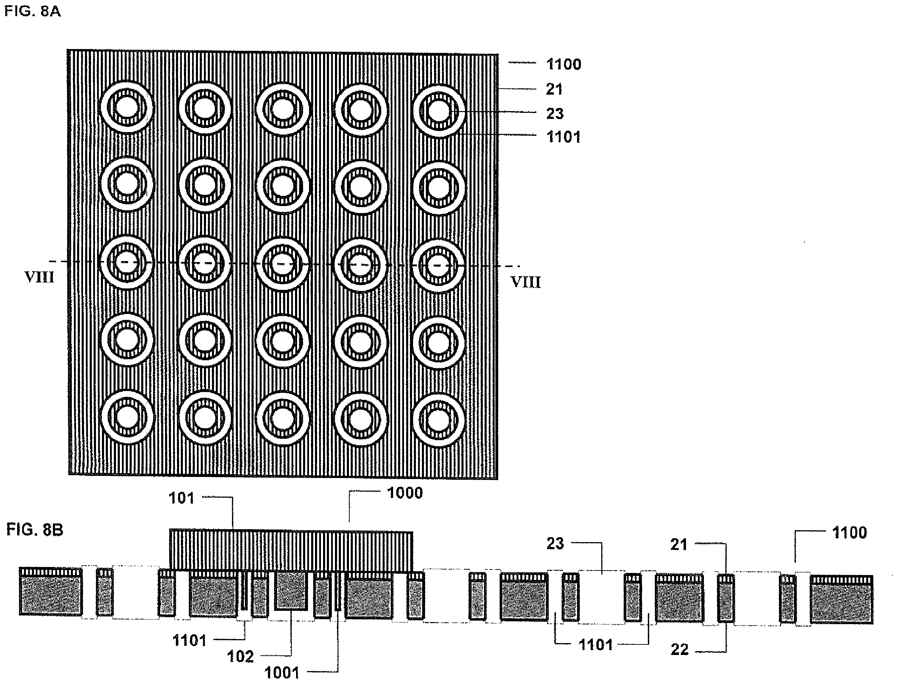

Ainsi, les figures 7 et 8 illustrent une première variante.

Les figures 7A (vue de dessus) et 7B (vue en coupe selon la ligne VII-VII sur la figure 7A) représentent la fiche 1000 du connecteur électrique.

Par rapport à la fiche 100 illustrée à la figure 2, la fiche 1000 comprend en plus une pièce annulaire 1001 formant une protubérance.

Les trois pièces 101, 102 et 1001 sont centrées autour d'un même axe.

La fiche 1000 conserve une symétrie de révolution autour d'un axe.

Les figures 8A (vue de dessus) et 8B (vue en coupe selon la ligne VIII-VIII sur la figure 8A) représentent l'embase 1100 du connecteur électrique, modifiée pour s'adapter à la fiche illustrée à la figure 7. Ainsi, par rapport à l'embase 20 illustrée à la figure 1, l'embase 1100 comprend en plus des trous annulaires 1101.

Chaque trou 23 est entouré d'un trou annulaire 1101, les deux trous étant centrés autour d'un même axe. L'ensemble formé par un trou cylindrique 23 et un trou annulaire 1101 possède donc une symétrie de révolution autour d'un même axe central.

La figure 8B représente la fiche 1000 une fois connectée à l'embase 1100.

L'un des trous cylindriques 23 accueille la protubérance 102, tandis que le trou annulaire 1101 correspondant accueille la protubérance 1001.

Par rapport au cas décrit à la figure 2B, la tenue mécanique de la connexion vis-à-vis des forces latérales est améliorée, car une protubérance supplémentaire (la protubérance 1001) est présente.

Avantageusement, le diamètre de la protubérance 102 est tel que la protubérance 102 ne peut pas être introduite dans un trou annulaire 1101.

Cette première variante est décrite en relation aux figures 1 et 2, mais elle s'applique également au connecteur décrit en relation avec les figures 3, 4 et 5, 6.

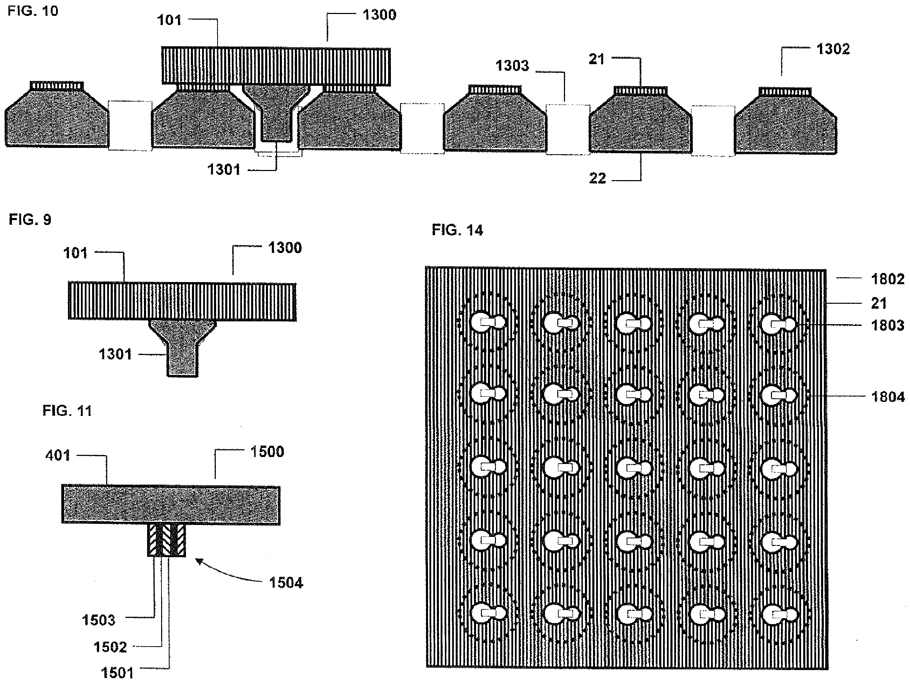

Les figures 9 et 10 illustrent une deuxième variante de réalisation du connecteur.

La figure 9 représente la fiche 1300 du connecteur électrique.

Contrairement à la protubérance 102 de la fiche 100 illustrée à la figure 2B, la protubérance 1301 de la fiche 1300 présente une forme évasée près de sa base. En d'autres termes, le diamètre de la protubérance diminue en s'éloignant de la base 101 de la fiche. La fiche 1300 conserve une symétrie de révolution autour d'un axe.

La figure 10 représente l'embase 1302 du connecteur électrique. Contrairement aux trous 23 de l'embase 20 illustrée à la figure 1, les trous 1303 de l'embase 1302 présentent une forme évasée près de la couche 21 de l'embase. En d'autres termes, les trous s'élargissent à proximité de la couche 21. Chaque trou 1303 conserve une symétrie de révolution autour d'un axe.

La figure 10 représente également la fiche 1300 une fois connectée à l'embase 1302. La forme évasée de la protubérance 1301 et des trous 1303 facilite la connexion, en guidant l'insertion de la protubérance 1301 dans un trou 1303.

Cette deuxième variante est décrite en relation aux figures 1 et 2, mais elle s'applique également au connecteur décrit en relation avec les figures 3, 4 et 5, 6.

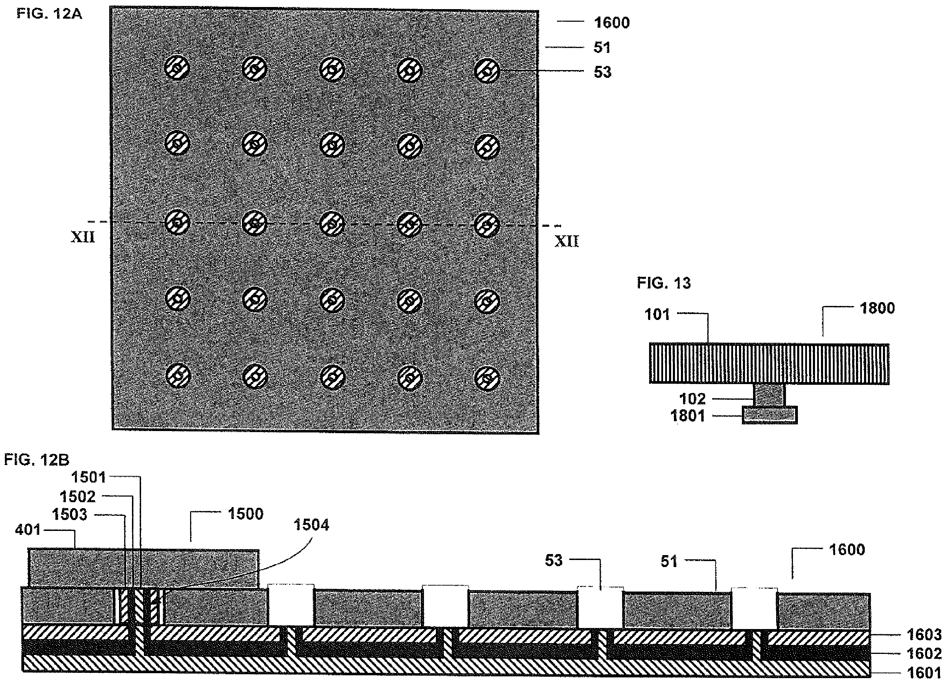

Les figures 11 et 12 illustrent une troisième variante de réalisation du connecteur.

La figure 11 représente la fiche 1500 du connecteur électrique.

Contrairement à la protubérance 402 de la fiche 400 illustrée à la figure 4B, la protubérance 1504 de la fiche 1500 permet de transmettre non pas un seul signal électrique mais deux signaux électriques.

En effet, la protubérance de la fiche 1500 est constituée d'une pièce cylindrique centrale 1501 ayant une fonction électrique et magnétique, d'une pièce annulaire 1502 ayant une fonction d'isolant électrique, et d'une pièce annulaire 1503 ayant une fonction électrique et magnétique. Les pièces 1501 et 1503, isolées électriquement par la pièce 1502, peuvent chacune transmettre un signal électrique différent.

Les quatre pièces 401, 1501, 1502 et 1503 sont centrées autour d'un même axe. La fiche 1500 conserve ainsi une symétrie de révolution autour d'un même axe.

Les figures 12 (vue de dessus) et 12B (vue en coupe selon la ligne XII-XII sur la figure 12A) représentent l'embase 1600 du connecteur électrique, modifiée pour s'adapter à la fiche illustrée à la figure 11.

Contrairement à la couche inférieure 52 de l'embase 50 illustrée à la figure 3, la couche inférieure de l'embase 1600 permet de transmettre non pas un seul signal électrique mais deux signaux électriques.

En effet, la couche inférieure de l'embase 1600 est constituée d'une couche 1601 jouant un rôle électrique et magnétique, d'une couche 1602 ayant une fonction d'isolant électrique, et d'une couche 1603 ayant une fonction électrique et magnétique. Les couches 1601 et 1603, isolées électriquement par la couche 1602, peuvent chacune transmettre un signal électrique différent.

La figure 12B représente également la fiche 1500, une fois connectée à l'embase 1600.

La couche 1601 établit un contact électrique avec la pièce 1501. Par ailleurs, la couche 1601 et la pièce 1501 exercent l'une sur l'autre une force magnétique attractive.

La couche 1603 établit un contact électrique avec la pièce 1503. Par ailleurs, la couche 1603 et la pièce 1503 exercent l'une sur l'autre une force magnétique attractive.

Cette troisième variante est décrite en relation avec les figures 3 et 4, mais elle s'applique également au connecteur décrit en référence aux figures 5 et 6.

Les figures 13 et 14 illustrent une quatrième variante de réalisation du connecteur selon l'invention.

La figure 13 représente la fiche 1800 du connecteur électrique.

Par rapport à la fiche 100 illustrée à la figure 2B, la fiche 1800 présente une forme s'élargissant en s'éloignant de la base. Plus particulièrement, la fiche 1800 illustrée à la figure 13 comprend une pièce cylindrique 1801 fixée à l'extrémité de la protubérance 102, le diamètre de la pièce cylindrique 1801 étant supérieur à celui de la pièce cylindrique 102. Les trois pièces 101, 102 et 1801 sont centrées autour d'un même axe. La fiche 1800 conserve donc une symétrie de révolution autour d'un même axe.

La figure 14 représente l'embase 1802 du connecteur électrique qui est du type illustré à la figure 1. Elle montre que les couches supérieure 21 et inférieure 22 de l'embase 1802 sont modifiées pour s'adapter à la fiche illustrée à la figure 13.

Les trous 1803 traversant la couche supérieure 21 ont une forme différente des trous 1804 traversant la couche inférieure 22. Ainsi, les trous 1803 sont formés d'un grand cylindre et d'un petit cylindre, alors que les trous 1804 sont simplement cylindriques. Les trous 1803 et 1804 sont alignés, comme représenté à la figure 14 (les pointillés sur cette figure indiquent la position des trous 1804 par rapport aux trous 1803).

Le diamètre de la pièce 102 est inférieur au diamètre du petit cylindre des trous 1803, et donc inférieur au diamètre du grand cylindre des trous 1803. Le diamètre de la pièce 1801 est supérieur au diamètre du petit cylindre des trous 1803, mais inférieur au diamètre du grand cylindre des trous 1803.

La protubérance de la fiche 1800 est insérée dans l'embase 1802, en passant d'abord par le grand cylindre d'un trou 1803, ensuite par le trou 1804 sous-jacent. Après cette insertion, la pièce 1801 se retrouve dans le trou 1804, à la verticale du grand cylindre du trou 1803. Dans cette position dite *« non verrouillée »,* la protubérance de la fiche 1800 empêche une déconnexion accidentelle due à des forces latérales, mais n'oppose aucune résistance à des forces verticales.

A partir de la position « *non verrouillée* », la protubérance de la fiche 1800 peut être déplacée latéralement vers la droite de manière à ce que la pièce 1801 reste dans le trou 1804 mais se retrouve à la verticale du petit cylindre du trou 1803. Dans cette position dite « *verrouillée* », la protubérance de la fiche 1800 empêche une déconnexion accidentelle due à des forces latérales et, de plus, oppose une résistance à des forces verticales. En effet, le diamètre de la pièce 1801 est supérieur au diamètre du petit cylindre du trou 1803.

Le connecteur électrique représenté aux figures 13 et 14 permet donc de renforcer la résistance à une déconnexion accidentelle, en plaçant la fiche dans une position « *verrouillée* »*.*

D'autres formes de trous 1803 sont envisageables pour assurer un mode *« verrouillé ».* Par exemple, les trous 1803 peuvent être formés d'un grand cylindre entouré d'une pluralité de petits cylindres.

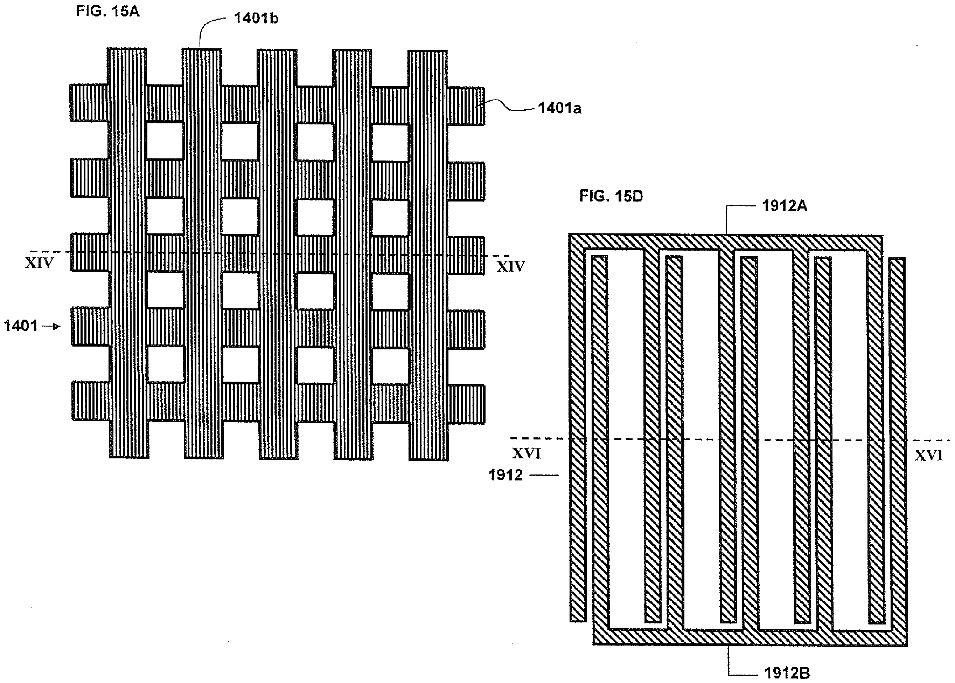

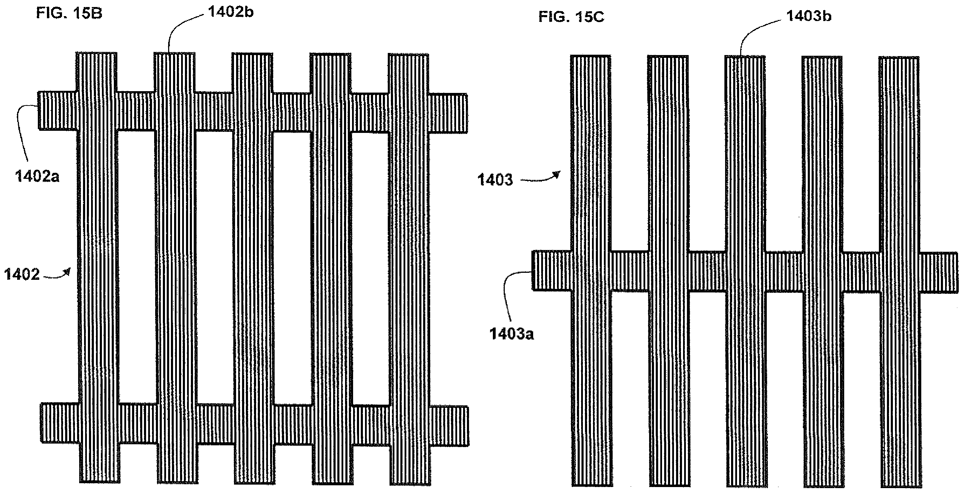

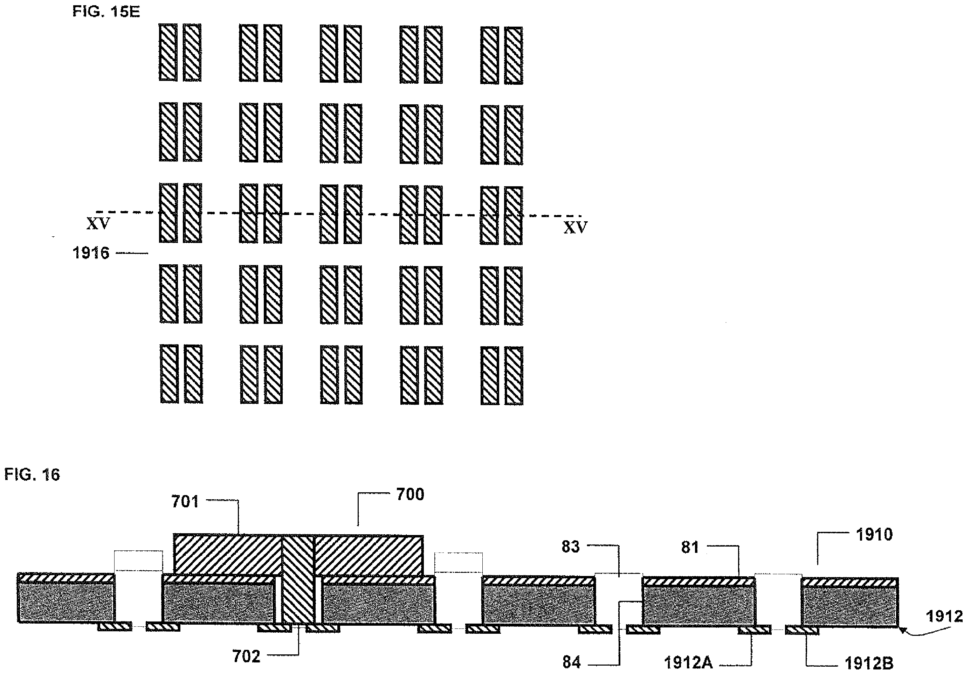

Les figures 15 et 16 illustrent une cinquième variante de réalisation d'un connecteur comportant une embase du type illustré aux figures 3 et 5, c'est-à-dire incluant une couche inférieure 52 ou 82 non traversée par des trous.

La figure 15A représente la couche inférieure 1401 de l'embase selon cette cinquième variante dont la vue en coupe selon la ligne XIV-XIV de la figure 15A est similaire à celle de l'embase 50 illustrée à la figure 3B ou la figure 5B. Contrairement à la couche inférieure 52 de l'embase 50 illustrée à la figure 3, la couche inférieure 1401 de cette embase n'est pas une couche continue, mais une couche en forme de grille : la flexibilité de la couche inférieure est donc améliorée. Cette grille est composée de bandes horizontales 1401a et de bandes verticales 1401b. Les trous 53 débouchent sur des zones pleines de la grille 1401.

La couche inférieure de l'embase peut prendre d'autres formes, comme celles illustrées sur les figures 15B et 15C.

Ainsi, la couche inférieure 1402 comporte autant de bandes verticales 1402b que la grille 1401 de la figure 15A mais seulement deux bandes horizontales 1402a.

La couche inférieure 1403 ne comporte qu'une seule bande horizontale 1403a et autant de bandes verticales 1403b que la grille 1401.

L'intérêt des couches 1402 et 1403 est d'augmenter encore la flexibilité mécanique de la couche inférieure de l'embase.

Ces modes de réalisation sont possibles à condition que le matériau formant la couche inférieure ait une conductivité électrique suffisamment élevée. En effet, dans ce cas, toutes les bandes horizontales sur la figure 15A ne sont pas forcément nécessaires pour conduire le courant électrique.

La figure 15D représente la couche inférieure 1912 de l'embase du connecteur électrique. Contrairement à la couche inférieure 82 de l'embase 80, illustrée à la figure 5, la couche inférieure 1912 de l'embase illustrée à la figure 15D n'est pas une couche continue, mais une couche constituée de deux pièces 1912A et 1912B distinctes et non connectées électriquement.

La figure 16 est une vue en coupe de l'embase selon la ligne XVI-XVI sur la figure 15D. Elle illustre que les trous 83 de l'embase débouchent sur des zones comprenant à la fois une partie de la pièce 1912A et une partie de la pièce 1912B. Dans le cas de l'exemple illustré à la figure 15D, les deux pièces 1912A et 1912B forment une structure inter-digitée, mais d'autres formes et structures sont envisageables.

La figure 16 représente la fiche 700 une fois connectée à l'embase 1910. Les deux pièces 1912A et 1912B établissent un contact électrique avec la protubérance 702a. De plus, la protubérance 702a permet de connecter électriquement la pièce 1912A à la pièce 1912B.

Ainsi, l'embase 1910 assure à la fois une fonction de connecteur et d'interrupteur. Ceci est particulièrement avantageux, comme cela sera expliqué dans la suite de la description.

Par ailleurs, le fait que la couche inférieure 1912 ne soit pas une couche continue peut avantageusement conférer à l'embase 1910 une plus grande flexibilité.

Dans l'exemple illustré à la figure 15D, la couche inférieure est constituée de deux pièces distinctes et non connectées électriquement.

On peut généraliser ce concept au cas d'un nombre plus élevé de pièces distinctes et non connectées électriquement : c'est par exemple le cas de la couche inférieure 1916, illustrée à la figure 15E, destinée à une autre embase dont la vue en coupe selon la ligne XV-XV de la figure 15E est similaire à celle de l'embase 1910 illustrée à la figure 16.

Cette embase peut permettre de détecter dans quel trou 83 est connectée la protubérance 702a. Ceci peut par exemple être obtenu par des moyens électroniques de mesure d'impédance.

C'est avantageux notamment pour des raisons de sécurité. Une électronique de contrôle peut alors faire en sorte que la puissance électrique soit dirigée seulement vers le trou 83 dans lequel est connectée la protubérance 702a, et non pas dans les autres trous de l'embase 1915.

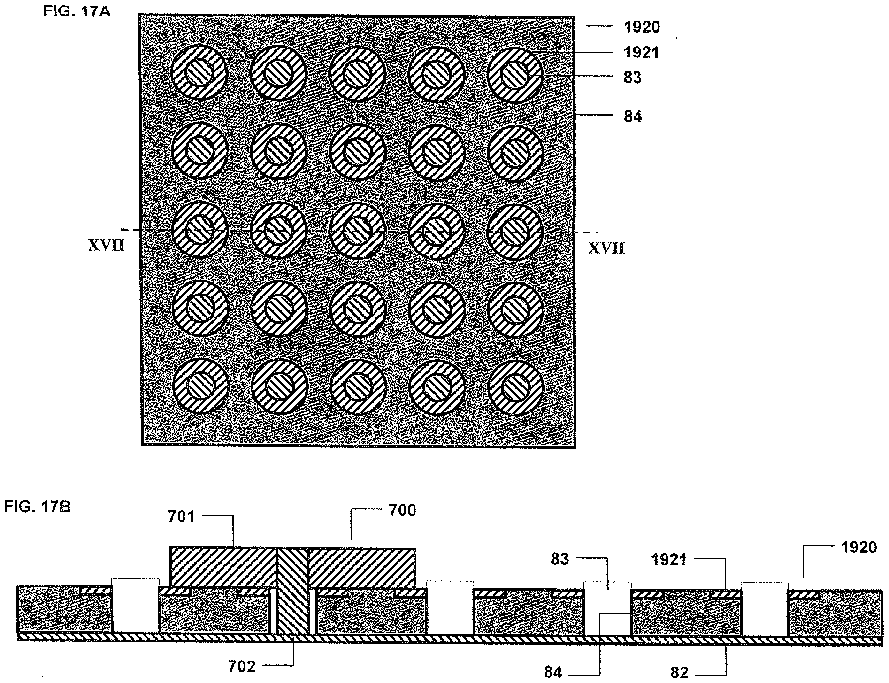

La figure 17A et la figure 17B (vue en coupe selon la ligne XVII-XVII de la figure 17A) représentent une sixième variante de réalisation de l'embase illustrée à la figure 5.

Contrairement à la couche supérieure 81 de l'embase 80, la couche supérieure 1921 de l'embase 1920 n'est pas une couche continue. La flexibilité de la couche supérieure est donc améliorée.

Dans le cas de l'exemple illustré à la figure 17, la couche supérieure 1921 est formée d'une pluralité d'anneaux non connectés mécaniquement, mais d'autres formes et structures sont envisageables (par exemple, une pluralité d'anneaux reliés entre eux par des bandes).

La figure 17B représente également la fiche 700 une fois connectée à l'embase 1920.

Dans une variante de réalisation non illustrée sur les figures, les protubérances des fiches remplissent une fonction élastique. Ainsi, elles peuvent être montées sur ressort (« *pogo pin* ») ou sur un matériau élastomère jouant le rôle de ressort. Alternativement, les protubérances des fiches peuvent être intégralement réalisées dans un matériau élastomère conducteur de l'électricité, par exemple un matériau élastomère chargé en éléments métalliques. Ceci peut permettre l'établissement d'un meilleur contact électrique.

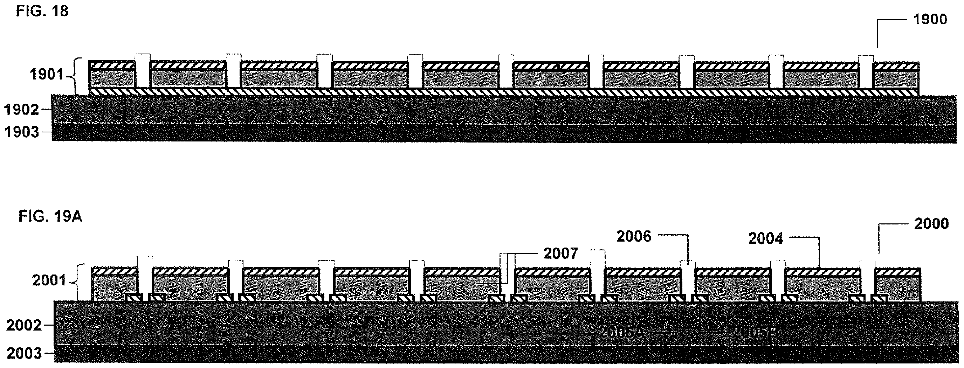

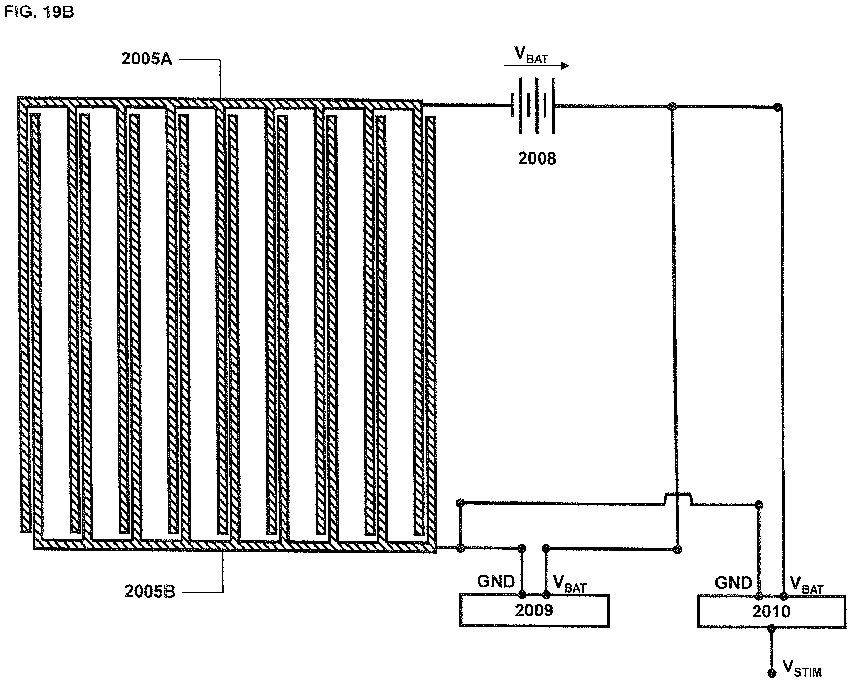

Les figures 18 et 19 représentent schématiquement comment le connecteur électrique selon l'invention peut être utilisé dans des dispositifs d'électrostimulation :
- une électrode cutanée auto-adhésive 1900, qui comprend une embase 1901 selon le troisième mode de réalisation de l'invention (figure 5B),
- un patch 2000 générant des impulsions, qui comprend une embase 2001 selon la cinquième variante de réalisation (figure 16).

Des connecteurs électriques selon d'autres modes de réalisation de l'invention peuvent également être utilisés dans de tels dispositifs d'électrostimulation.

La figure 18 montre que l'électrode cutanée 1900 comprend un substrat conducteur de l'électricité 1902, par exemple un polymère chargé de noir de carbone ou d'éléments métalliques, ou un matériau textile conducteur, et des couches d'hydrogel 1903, destinées à établir un contact électrique avec la peau de l'utilisateur.

L'électrode cutanée 1900 comprend sur sa face supérieure une embase 1901 selon le troisième mode de réalisation de l'invention. L'embase 1901 permet de connecter l'électrode cutanée 1900 à un fil électrique comprenant une fiche.

La figure 19A montre que le patch 2000 comprend une partie principale 2002 qui contient notamment des composants électroniques et une source d'énergie électrique.

Il comprend sur sa face inférieure des couches d'hydrogel 2003, destinées à établir un contact électrique avec la peau de l'utilisateur et, sur sa face supérieure, une embase 2001 selon la cinquième variante de l'invention.

L'embase 2001 permet de connecter le patch 2000 à un fil électrique comprenant une fiche.

Elle peut également jouer le rôle de couche d'encapsulation supérieure pour le patch 2000.

L'embase 2001 comprend une couche supérieure 2004 jouant un rôle magnétique, une couche intermédiaire 2007 jouant un rôle mécanique, et une couche inférieure 2005 jouant un rôle électrique.

La couche inférieure 2005 est constituée de deux pièces 2005A et 2005B, distinctes et non connectées électriquement. Des trous 2006 débouchent sur des zones comprenant à la fois une partie de la pièce 2005A et une partie de la pièce 2005B.

La figure 19B est un schéma électrique du patch 2000.

La pièce 2005A est connectée au pôle négatif d'une source d'énergie électrique 2008. La pièce 2005B est connectée aux masses (GND) des circuits électroniques 2009 et 2010. Le pôle positif de la source d'énergie électrique 2008 est connecté aux alimentations (V_{BAT}) des circuits électroniques 2009 et 2010. La sortie (V_{STIM}) du circuit électronique 2010 est connectée aux couches d'hydrogel 2003.

Le circuit électronique 2010 a pour rôle de générer des impulsions d'électrostimulation.

Le circuit électronique 2009 peut avoir plusieurs rôles, en particulier :
- avertir l'utilisateur lorsque le patch 2000 est allumé, par exemple grâce un signal visuel ou sonore,
- communiquer avec l'utilisateur, par exemple par ondes radio, c'est-à-dire transmettre des informations à l'utilisateur ou recevoir des informations provenant de l'utilisateur,
- piloter le circuit électronique 2010, c'est-à-dire ordonner le démarrage ou l'arrêt d'un certain programme d'électrostimulation, caractérisé notamment par l'amplitude, la fréquence et la durée des impulsions.

Au cours de l'utilisation du patch 2000, deux cas peuvent se présenter :

### 1) Aucune fiche n'est connectée à l'embase 2001 du patch 2000.

Dans ce cas, l'interrupteur formé par les deux pièces 2005A et 2005B est dans un état ouvert. En effet, les deux pièces 2005A et 2005B ne sont pas connectées électriquement. Les circuits électroniques 2009 et 2010 ne sont donc pas alimentés par la source d'énergie électrique 2008. Autrement dit, le patch 2000 est éteint et ne consomme pas d'énergie.

### 2) Une fiche est connectée à l'embase 2001 du patch 2000.

Dans ce cas, l'interrupteur formé par les deux pièces 2005A et 2005B est dans un état fermé. En effet, la protubérance de la fiche, qui est insérée dans un trou 2006, permet de connecter électriquement la pièce 2005A à la pièce 2005B. Les circuits électroniques 2009 et 2010 sont donc alimentés par la source d'énergie électrique 2008. Autrement dit, le patch 2000 est allumé et consomme de l'énergie provenant de la source 2008.

Dans ce cas, comme mentionné plus haut, le circuit électronique 2009 peut :
- avertir l'utilisateur du fait que le patch 2000 est allumé,
- transmettre des informations à l'utilisateur ou se tenir prêt à recevoir des informations provenant de l'utilisateur,
- piloter le circuit électronique 2010.

Ainsi, dans le premier cas (interrupteur ouvert, patch éteint), aucun courant d'électrostimulation ne peut circuler dans le corps de l'utilisateur.

Cependant, dans le deuxième cas (interrupteur fermé, patch allumé), un courant d'électrostimulation ne circule pas nécessairement dans le corps de l'utilisateur. En effet, pour qu'un courant d'électrostimulation circule dans le corps de l'utilisateur, il faut d'une part, que le chemin électrique d'électrostimulation soit établi, comme décrit plus bas, et d'autre part, que le circuit électronique 2009 autorise le circuit électronique 2010 à démarrer un programme d'électrostimulation.

L'avantage de l'embase 2001 selon la cinquième variante de l'invention est d'assurer une fonction à la fois de connecteur et d'interrupteur. Ceci permet de ne pas à avoir à intégrer un interrupteur conventionnel dans le patch 2000. Ceci est intéressant notamment si l'on désire que le patch 2000 reste un objet fin et flexible.

La figure 20A représente un utilisateur (vue de dos) portant, dans le bas du dos, l'électrode cutanée auto-adhésive 1900 et le patch 2000.

L'électrode cutanée auto-adhésive 1900 est connectée à la peau de l'utilisateur via les couches d'hydrogel 1903.

La sortie (V_{STIM}) du circuit électronique 2010 du patch 2000 est connectée à la peau de l'utilisateur via les couches d'hydrogel 2003.

La figure 20A montre que l'électrode cutanée auto-adhésive 1900 et le patch 2000 sont interconnectés via une bande élastique 2100.

Cette bande 2100 est illustrée vue de dessus sur la figure 20B.

Elle comprend une piste 2103 conductrice de l'électricité, interconnectant deux fiches 2101 et 2102, lesquelles sont destinées à être connectées avec les embases 1901 et 2001.

La piste 2103 est par exemple une bicouche métal/polymère, fixée sur la bande élastique 2100. La piste 2103 peut présenter une forme en serpentin, comme décrit par exemple à la figure 3 du document [R. Carta et al., Design and implementation of advanced systems in a flexible-stretchable technology for biomédical applications, Sensors and Actuators A 156 (2009) 79-87]. De cette manière, la bande élastique 2100 peut être étirée sans risque d'endommagement de la piste 2103.

La fiche 2101 sur la bande élastique 2100 est connectée à l'embase 1901 de l'électrode cutanée 1900. La fiche 2102 sur la bande élastique 2100 est connectée à l'embase 2001 du patch 2000.

Ainsi, le pôle négatif de la source d'énergie 2008 est connecté à la peau de l'utilisateur via les couches d'hydrogel 1903. De cette manière, un chemin électrique d'électrostimulation est établi : un courant électrique peut circuler dans le corps de l'utilisateur entre les couches d'hydrogel 2003 (qui sont au potentiel électrique V_{STIM}) et les couches d'hydrogel 1903 (qui sont au potentiel électrique du pôle négatif de la source d'énergie 2008).

Les avantages du connecteur électrique selon l'invention utilisé dans l'électrode cutanée 1900 et le patch 2000 sont les suivants :
- les embases 1901 et 2001 peuvent être fines voire flexibles, ce qui est confortable pour l'utilisateur ;
- la connexion entre l'électrode cutanée 1900 et le patch 2000, via les embases 1901 et 2001 et les fiches 2101 et 2102, peut être effectuée rapidement sans exercer de pression sur la peau de l'utilisateur et sans contrôle visuel ;
- l'embase 1901, respectivement 2001, présente un grand nombre de zones de connexion potentielles (les trous de l'embase), ces zones de connexion couvrant une grande partie de la surface supérieure de l'électrode cutanée 1900 (respectivement du patch 2000). Ainsi, pour une longueur donnée de bande élastique 2100, l'utilisateur se voit offert une grande liberté sur la distance séparant l'électrode cutanée 1900 du patch 2000. De plus, l'utilisateur peut facilement connecter la bande élastique 2100 de manière à ce qu'elle ne soit ni trop tendue (dans ce cas le risque de déconnexion accidentelle serait accru, et la bande élastique 2100 serait une source de gêne car elle comprimerait le corps de l'utilisateur), ni trop lâche (dans ce cas la bande élastique 2100 serait une source de gêne car elle « *pendrait dans* /*e vide* »)*.*

La bande élastique 2100 peut comprendre une première languette permettant à l'utilisateur de déconnecter facilement la fiche 2101 de l'embase 1901, et une deuxième languette permettant à l'utilisateur de déconnecter facilement la fiche 2102 de l'embase 2001.

Enfin, la bande élastique 2100 peut être intégrée dans un vêtement.

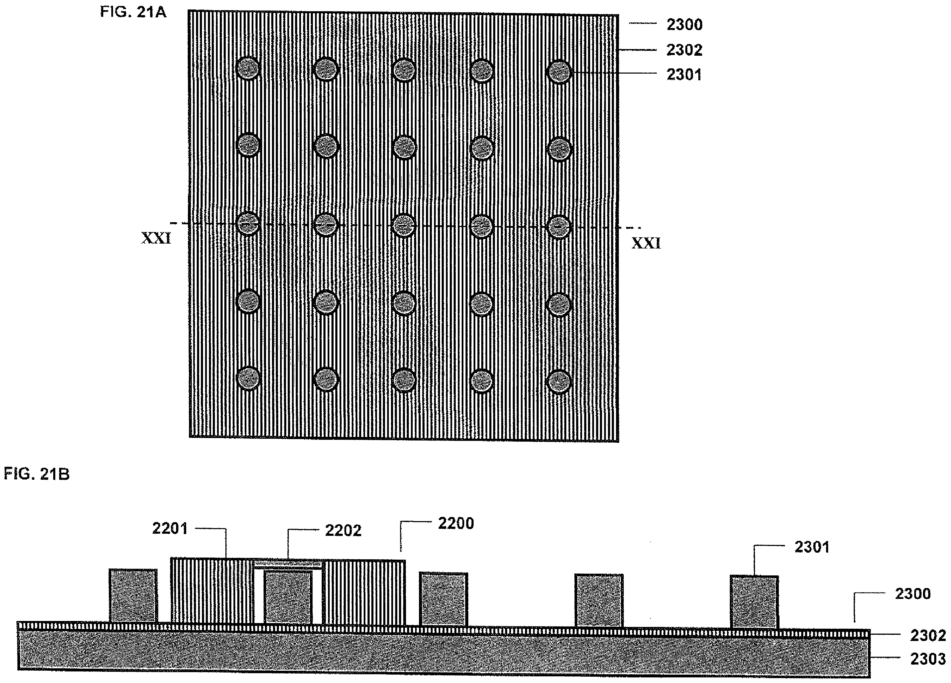

Les figures 21 à 26 illustrent plusieurs formes de réalisation d'un connecteur selon l'invention comportant une embase mâle et une fiche femelle comportant une cavité.

L'embase comprend un empilement de couches, avantageusement fines voire flexibles, et une pluralité de protubérances.

Ainsi, les figures 21 et 22 illustrent une première forme de réalisation d'un tel connecteur, dans laquelle, l'embase comprend :
- une pluralité de protubérances,
- une couche intermédiaire ayant une fonction électrique et magnétique, sur laquelle sont réalisées les protubérances,
- une couche inférieure ayant une fonction mécanique.

Une variante de cette première forme de réalisation consiste en ce que la couche intermédiaire est supprimée, la couche inférieure remplissant également une fonction électrique et une éventuelle fonction magnétique.

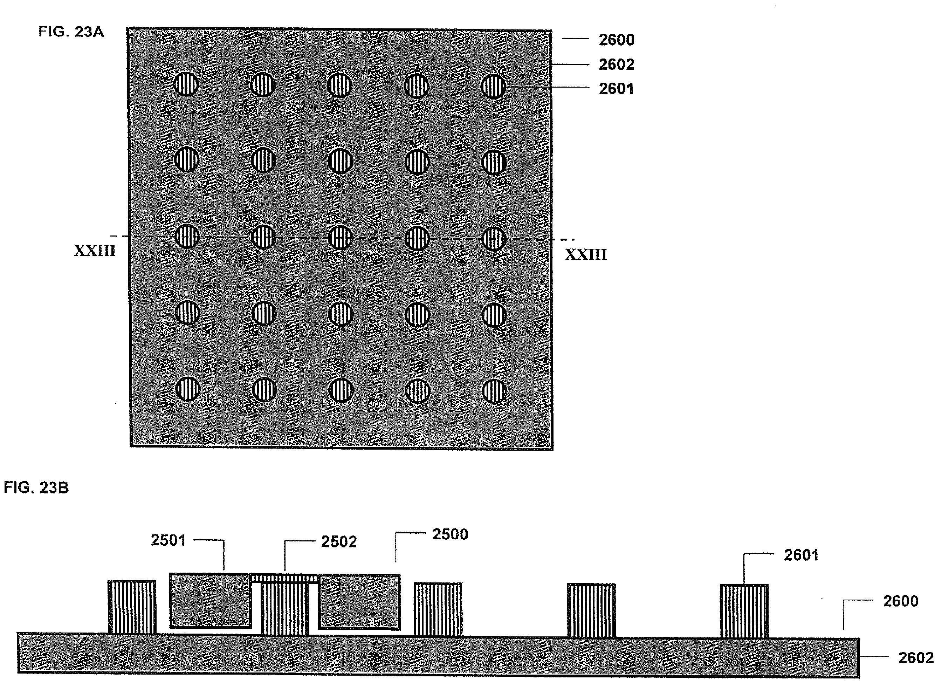

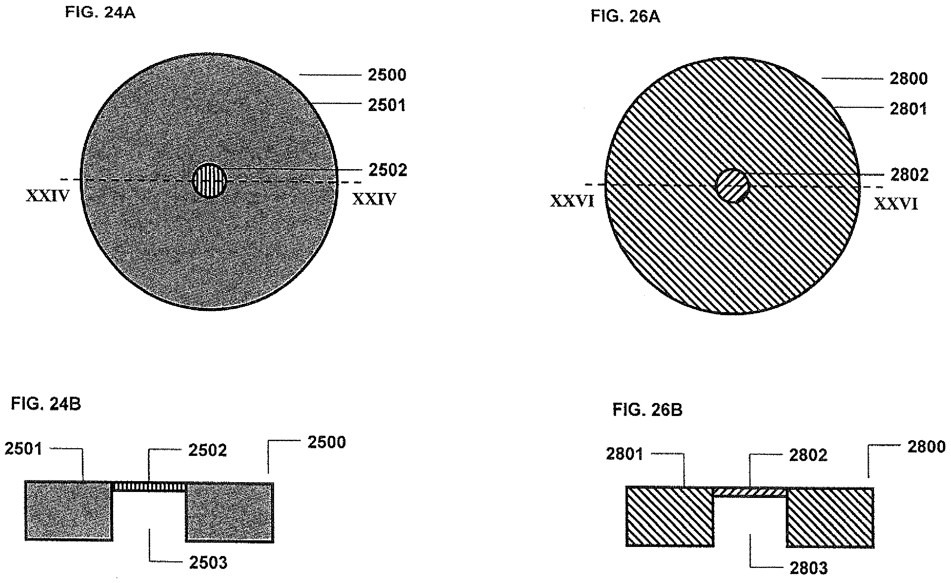

Les figures 23 et 24 illustrent une deuxième forme de réalisation d'un tel connecteur, dans laquelle, l'embase comprend :
- une pluralité de protubérances ayant une fonction électrique et magnétique,
- une couche inférieure ayant une fonction mécanique, sur laquelle sont formées les protubérances.

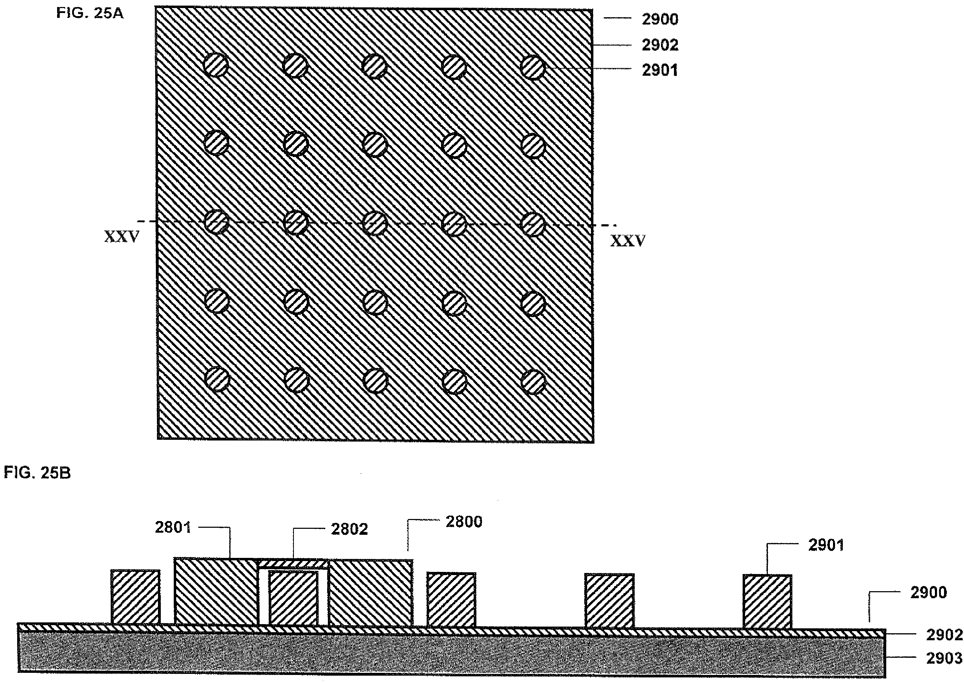

Les figures 25 et 26 illustrent une troisième forme de réalisation d'un tel connecteur, dans laquelle, l'embase comprend :
- une pluralité de protubérances ayant une fonction magnétique,
- une couche intermédiaire ayant une fonction électrique, sur laquelle sont formées les protubérances,
- une couche inférieure ayant une fonction mécanique.

Une variante de cette troisième forme de réalisation (non illustrée sur les figures) consiste en ce que les protubérances assurent une fonction électrique et la couche intermédiaire assure une fonction magnétique.

Dans chacune de ces formes de réalisation :
- L'embase comprend soit des protubérances ayant une fonction électrique, soit une couche ayant une fonction électrique. Les éléments de l'embase ayant une fonction électrique (protubérance ou couche) sont destinés à établir un contact électrique avec la pièce de la fiche ayant une fonction électrique.
Les éléments de l'embase ayant une fonction électrique sont formés dans un matériau conducteur de l'électricité.
Si l'embase comprend une couche ayant une fonction électrique, cette couche est de préférence fine voire flexible.
- L'embase comprend soit des protubérances ayant une fonction magnétique, soit une couche ayant une fonction magnétique. Les éléments de l'embase ayant une fonction magnétique (protubérance ou couche) et la pièce de la fiche ayant une fonction magnétique sont destinés à exercer entre eux une force magnétique attractive.
Les éléments de l'embase ayant une fonction magnétique sont formés dans un matériau ferromagnétique ou un matériau aimanté.
Si l'embase comprend une couche ayant une fonction magnétique, cette couche est de préférence fine voire flexible.
- La couche de l'embase ayant une fonction mécanique sert de support à une pluralité de protubérances. Elle est de préférence fine voire flexible.
L'une quelconque des protubérances est destinée à être insérée dans la cavité de la fiche et à empêcher ainsi une déconnexion accidentelle due à des forces latérales, c'est-à-dire des forces dans le plan de l'embase.
La hauteur des protubérances est suffisamment importante pour qu'elles assurent bien leur fonction de maintien latéral de la fiche. Avantageusement, la hauteur des protubérances est comprise entre 0.1 mm et 5 mm, de préférence entre 0.5 mm et 2 mm.
- La densité surfacique de protubérances sur l'embase est suffisamment élevée pour permettre une connexion rapide sans contrôle visuel.
Ceci est facilité par l'action des forces magnétiques. Cependant, cette densité de protubérances permet d'obtenir une connexion sans contrôle visuel, même en l'absence de pièces remplissant une fonction magnétique à l'intérieur du connecteur.
Cependant, en présence de pièces remplissant une fonction magnétique, on constate qu'un petit déplacement latéral de la fiche à proximité de la surface de l'embase suffit pour que les forces magnétiques entrent en action et assemblent les deux parties du connecteur, en provoquant l'insertion de l'une des protubérances de l'embase dans la cavité de la fiche.
Autrement dit, la forte densité surfacique de zones de connexion potentielles (les protubérances de l'embase), combinée à l'action des forces magnétiques, facilite une connexion rapide sans contrôle visuel. Avantageusement, le diamètre des protubérances (en considérant des protubérances cylindriques) est compris entre 0.1 mm et 10 mm, de préférence entre 0.5 mm et 5 mm.
Avantageusement, la distance centre à centre entre deux protubérances voisines est comprise entre 1.5 fois le diamètre et 10 fois le diamètre, de préférence entre 1.5 fois le diamètre et 5 fois le diamètre.
Avantageusement, les protubérances de l'embase sont réparties de façon uniforme sur la surface de l'embase.
Dans le cas où l'embase est destinée à un dispositif porté sous la forme d'un patch, la somme des surfaces occupées par les protubérances correspond avantageusement à plus de 1% de la surface totale du patch et, de préférence, à plus de 5% de la surface totale du patch.

Par ailleurs, dans toutes ces formes de réalisation, la fiche comprend :
- une cavité,
- une pièce ayant une fonction électrique (c'est-à-dire une pièce formée dans un matériau conducteur de l'électricité),
- une pièce ayant une fonction magnétique (c'est-à-dire une pièce formée dans un matériau ferromagnétique ou un matériau aimanté).

Une même pièce dans la fiche peut assurer à la fois une fonction électrique et magnétique.

En référence tout d'abord aux figures 21 et 22, les figures 21A (vue de dessus) et 21B (vue en coupe selon la ligne XXI-XXI sur la figure 21A) représentent l'embase 2300 du connecteur électrique.

L'embase 2300 comprend une pluralité de protubérances 2301 (formant ici un réseau périodique), une couche intermédiaire 2302 ayant une fonction électrique et magnétique, et une couche inférieure 2303 ayant une fonction mécanique.

Les protubérances 2301 sont par exemple en métal. La couche intermédiaire 2302 est par exemple une feuille flexible en acier inoxydable magnétique. La couche inférieure 2303 est par exemple une couche flexible en polymère ou en matériau textile.

Les figures 22A (vue de dessus) et 22B (vue en coupe selon la ligne XXII-XXII sur la figure 22A) représentent la fiche 2200 du connecteur électrique.

La fiche 2200 comprend une pièce cylindrique 2201 ayant une fonction électrique et magnétique définissant une cavité 2203 qui est fermée par une pièce cylindrique 2202 formant la base de la fiche.

Les deux pièces cylindriques 2201 et 2202 sont centrées autour d'un même axe.

La pièce 2201 est par exemple une pièce en aluminium-nickel-cobalt, ou samarium-cobalt, ou néodyme-fer-bore, qui sont des matériaux aimantés et conducteurs de l'électricité. La pièce 2202 est par exemple une pièce en métal.

La figure 21B représente également la fiche 2200 une fois connectée à l'embase 2300.

L'une des protubérances 2301 est insérée dans la cavité 2203, ce qui empêche une déconnexion accidentelle due à des forces latérales, c'est-à-dire des forces dans le plan de l'embase 2300.

La couche intermédiaire 2302 établit un contact électrique avec la pièce 2201. La couche intermédiaire 2302 et la pièce 2201 exercent l'une sur l'autre une force magnétique attractive.

En référence maintenant aux figures 23 et 24, les figures 23A (vue de dessus) et 23B (vue en coupe selon la ligne XXIII-XVIII sur la figure 23A) représentent l'embase 2600 du connecteur électrique.

L'embase 2600 comprend une pluralité de protubérances 2601 (formant ici un réseau périodique) ayant une fonction électrique et magnétique, et une couche inférieure 2602 ayant une fonction mécanique.

Les protubérances 2601 sont par exemple en acier inoxydable magnétique. La couche inférieure 2602 est par exemple une couche flexible en polymère ou en matériau textile.

Les figures 24A (vue de dessus) et 24B (vue en coupe selon la ligne XXIV-XXIV sur la figure 24A) représentent la fiche 2500 du connecteur électrique.

La fiche 2500 comprend une pièce cylindrique 2501 et une pièce cylindrique 2502 formant la base de la fiche et ayant une fonction électrique et magnétique.

La pièce 2502 forme avec la pièce 2501 une cavité 2503. Les deux pièces cylindriques 2501 et 2502 sont centrées autour d'un même axe.

La pièce 2501 est par exemple une pièce en métal. La pièce 2502 est par exemple une pièce en aluminium-nickel-cobalt, ou samarium-cobalt, ou néodyme-fer-bore, qui sont des matériaux aimantés et conducteurs de l'électricité.

La figure 23B représente également la fiche 2500 une fois connectée à l'embase 2600.

L'une des protubérances 2601 est insérée dans la cavité 2503, ce qui empêche une déconnexion accidentelle due à des forces latérales, c'est-à-dire des forces dans le plan de l'embase 2600.

La protubérance 2601 établit un contact électrique avec la pièce 2502. La protubérance 2601 et la pièce 2502 exercent l'une sur l'autre une force magnétique attractive.

En référence enfin aux figures 25 et 26, les figures 25A (vue de dessus) et 25B (vue en coupe selon la ligne XXV-XXV sur la figure 25A) représentent l'embase 2900 du connecteur électrique.

L'embase 2900 comprend une pluralité de protubérances 2901 (formant ici un réseau périodique) ayant une fonction magnétique, une couche intermédiaire 2902 ayant une fonction électrique, et une couche inférieure 2903 ayant une fonction mécanique.

Les protubérances 2901 sont par exemple en acier inoxydable magnétique. La couche intermédiaire 2902 est par exemple une feuille flexible en cuivre. La couche inférieure 2903 est par exemple une couche flexible en polymère ou en matériau textile.

Les figures 26A (vue de dessus) et 26B (vue en coupe selon la ligne XXVI-XXVI sur la figure 26A) représentent la fiche 2800 du connecteur électrique.

La fiche 2800 comprend une pièce cylindrique 2801 ayant une fonction électrique, et une autre pièce cylindrique 2802 formant la base de la fiche et ayant une fonction magnétique, La pièce 2802 forme avec la pièce 2801 une cavité 2803. Les deux pièces cylindriques 2801 et 2802 sont centrées autour d'un même axe.

La pièce 2801 est par exemple une pièce en métal. La pièce 2802 est par exemple une pièce en ferrite, ou aluminium-nickel-cobalt, ou samarium-cobalt, ou néodyme-fer-bore, qui sont des matériaux aimantés.

La figure 25B représente également la fiche 2800 une fois connectée à l'embase 2900. L'une des protubérances 2901 est insérée dans la cavité 2803, ce qui empêche une déconnexion accidentelle due à des forces latérales, c'est-à-dire des forces dans le plan de l'embase 2900. La couche intermédiaire 2902 établit un contact électrique avec la pièce 2801. La protubérance 2901 et la pièce 2802 exercent l'une sur l'autre une force magnétique attractive.

Dans toute la description qui précède, le connecteur selon l'invention comporte une fiche. Cependant, l'invention n'est pas limitée à ce mode de réalisation. Le connecteur pourrait comporter plusieurs fiches destinées à être connectées dans la même embase. Le nombre de fiches sera cependant strictement inférieur au nombre de moyens de connexion prévus dans l'embase.

Les signes de référence insérés après les caractéristiques techniques figurant dans les revendications ont pour seul but de faciliter la compréhension de ces dernières et ne sauraient en limiter la portée.

## Revendications

1. Connecteur électrique, notamment pour un dispositif médical destiné à être fixé sur la peau d'un utilisateur, comprenant une embase destinée à être solidaire du dispositif et une fiche destinée à être solidaire d'un conducteur électrique, **caractérisé en ce que** la fiche (100, 110, 120, 400, 410, 420, 700, 2200, 2500, 2800) comprend un unique moyen de connexion (102, 402, 702a, 2203, 2503, 2803) et l'embase (20, 50, 80, 2300, 2600, 2900) comprend une pluralité de moyens de connexion (23, 53, 83, 2301, 2601, 2901), chacun d'eux étant adaptés pour coopérer avec le moyen de connexion de la fiche pour assurer la connexion entre l'embase et la fiche.

2. Connecteur selon la revendication 1, **caractérisé en ce que** la distance centre à centre entre deux moyens de connexion de l'embase adjacents est comprise entre 1,5 et 10 fois la section de ces moyens de connexion.

3. Connecteur selon la revendication 1 ou 2, **caractérisé en ce que** les moyens de connexion de l'embase sont répartis de façon uniforme sur la surface de l'embase.

4. Connecteur selon l'une des revendications 1 à 3, **caractérisé en ce qu'**il comporte des moyens magnétiques à la fois sur la fiche et l'embase.

5. Connecteur selon l'une des revendications 1 à 4, **caractérisé en ce que** l'embase (20, 50, 80) comporte une pluralité de trous (23, 53, 83) et la fiche (100, 110, 120, 400, 410, 420, 700) comporte une protubérance (102, 402, 413, 423, 702a).

6. Connecteur selon la revendication 5, **caractérisé en ce que** l'embase (20, 50, 80) est constituée par un empilement d'au moins deux couches, l'une d'elles remplissant au moins une fonction de support mécanique et lesdites au moins deux couches remplissant également une fonction électrique et éventuellement une fonction magnétique, les trous (23, 53, 83) de l'embase traversant au moins la couche remplissant la fonction de support mécanique, et la fiche (100, 110, 120, 400, 410, 420, 700) comprenant une base et une protubérance (102, 402, 413, 423, 702a) remplissant une fonction électrique et éventuellement une fonction magnétique.

7. Connecteur selon la revendication 6, **caractérisé en ce que** l'empilement constituant l'embase (20) comprend une couche (22) remplissant une fonction de support mécanique et une couche (21) remplissant une fonction électrique et une éventuelle fonction magnétique, les trous (23) traversant l'empilement, la fonction électrique et l'éventuelle fonction magnétique de la fiche étant remplie(s) par la base (101, 110, 120).

8. Connecteur selon la revendication 6, **caractérisé en ce que** l'empilement constituant l'embase (50) comprend une couche (52) remplissant une fonction électrique et une couche (51) remplissant une fonction mécanique, la couche (52) remplissant une fonction électrique pouvant remplir également une fonction magnétique, les trous (53) traversant uniquement la couche (51) remplissant une fonction mécanique, la fonction électrique et éventuellement magnétique de la fiche (400, 410, 420) étant remplie par la protubérance (402, 413, 423).

9. Connecteur selon la revendication 6, **caractérisé en ce que** l'empilement constituant l'embase (80) comprend successivement une couche (82) remplissant une fonction électrique, une couche (84) remplissant une fonction mécanique et éventuellement une couche (81) remplissant une fonction magnétique, les trous (83) traversant la couche remplissant une fonction mécanique et, lorsqu'elle est présente, la couche remplissant une fonction magnétique, l'éventuelle fonction magnétique de la fiche (700) étant remplie par la base (701) et la fonction électrique de la fiche (700) étant remplie par la protubérance (702a).

10. Connecteur selon la revendication 9, **caractérisé en ce que** la couche (84) remplissant une fonction mécanique remplit également une fonction magnétique.

11. Connecteur selon l'une des revendications 5 à 10, **caractérisé en ce que** la fiche (1000) comprend une pièce annulaire (1001) en saillie sur la base (101) et s'étendant autour de la protubérance (102), l'embase comprenant autour de chaque trou, une ouverture annulaire (1101) pour recevoir la pièce annulaire en saillie de la fiche.

12. Connecteur selon l'une des revendications 5 à 11, **caractérisé en ce que** la fiche (1300) comporte une protubérance (1301) présentant une forme évasée se rétrécissant en s'éloignant de la base (101), les trous de l'embase comportant une forme correspondante pour recevoir cette protubérance.

13. Connecteur selon l'une des revendications 5 à 11, **caractérisé en ce que** la protubérance (102) de la fiche (1800) présente une forme évasée s'élargissant en s'éloignant de la base (101), les trous de l'embase présentant une forme correspondante pour recevoir cette protubérance.

14. Connecteur selon l'une des revendications 8 à 13, **caractérisé en ce que** la protubérance (1504) de la fiche (1500) comprend deux pièces (1501, 1503) remplissant une fonction électrique et éventuellement une fonction magnétique, séparées par un isolant (1502), la couche de l'embase qui remplit une fonction électrique étant conçue pour transmettre deux signaux électriques.

15. Connecteur selon l'une des revendications 8 à 13, **caractérisé en ce que** la couche de l'embase qui n'est pas traversée par les trous est une couche discontinue, se présentant notamment sous la forme d'une grille (1401) ou de pièces indépendantes (1912A, 1912B, 1916) et non connectées électriquement.

16. Connecteur selon l'une des revendications 7, 9 à 15, **caractérisé en ce qu'**une couche (1921) traversée par les trous est une couche discontinue.

17. Connecteur selon l'une des revendications 5 à 16, **caractérisé en ce que** la protubérance de la fiche remplit une fonction élastique.

18. Connecteur selon l'une des revendications 6 à 17, **caractérisé en ce que** la couche (21, 52, 82) remplissant une fonction électrique est constituée de deux pièces (1912A, 1912B) distinctes et non connectées électriquement, la protubérance (402, 413, 423 ; 702a) ou la base (100, 110, 120) de la fiche permettant de connecter électriquement ces deux pièces lorsque la fiche est connectée à l'embase.

19. Connecteur selon l'une des revendications 1 à 4, **caractérisé en ce que** l'embase (2300, 2600, 2900) comporte une pluralité de protubérances (2301, 2601, 2901) et la fiche (2200, 2500, 2800) comporte une cavité (2203, 2503, 2803).

20. Connecteur selon la revendication 19, **caractérisé en ce que** l'embase (2300, 2600, 2900) comprend au moins une couche remplissant une fonction de support mécanique et la pluralité de protubérances est réalisée directement sur la couche de support mécanique ou sur une couche intermédiaire, les protubérances, la couche remplissant une fonction de support mécanique ou la couche intermédiaire remplissant une fonction électrique et éventuellement une fonction magnétique, et la fiche (2200, 2500, 2800) comprend une pièce cylindrique définissant la cavité qui est fermée par une base, la fonction électrique de la fiche étant remplie par la pièce cylindrique ou la base et l'éventuelle fonction magnétique de la fiche étant remplie par la pièce cylindrique ou la base.

21. Connecteur selon la revendication 20, **caractérisé en ce que** l'embase (2300) comprend sur la couche (2303) remplissant une fonction de support mécanique, une éventuelle couche intermédiaire (2302), la fonction électrique et l'éventuelle fonction magnétique étant remplie(s) par la couche (2303) ou la couche intermédiaire (2302), les protubérances (2301) étant réalisées sur la couche (2303) ou sur la couche intermédiaire (2302), la fonction électrique et l'éventuelle fonction magnétique de la fiche (2200) étant remplie(s) par la pièce cylindrique (2201).

22. Connecteur selon la revendication 20, **caractérisé en ce que** l'embase (2600) comprend, sur la couche (2602) remplissant une fonction de support mécanique, des protubérances (2601) réalisées directement sur cette couche de support mécanique, ces protubérances remplissant une fonction électrique et éventuellement une fonction magnétique, la fonction électrique et l'éventuelle fonction magnétique de la fiche (2500) étant remplie(s) par la base (2502).

23. Connecteur selon la revendication 20, **caractérisé en ce que** l'embase (2900) comprend, sur la couche remplissant une fonction de support mécanique, une couche intermédiaire (2902) remplissant une fonction électrique, les protubérances (2901) étant réalisées sur cette couche intermédiaire et remplissant éventuellement une fonction magnétique, la pièce cylindrique (2801) de la fiche remplissant une fonction électrique et sa base (2802) remplissant éventuellement une fonction magnétique.

24. Connecteur selon la revendication 23, **caractérisé en ce que** les protubérances de l'embase remplissent une fonction électrique et la couche intermédiaire remplit éventuellement une fonction magnétique.

25. Connecteur selon l'une des revendications 1 à 24, **caractérisé en ce qu'**il comporte une pluralité de fiches dont le nombre est strictement inférieur à celui des moyens de connexion.

26. Dispositif médical destiné à être fixé sur la peau d'un utilisateur, comprenant un connecteur électrique selon l'une des revendications 1 à 25.

27. Dispositif selon la revendication 26, dans lequel les moyens de connexion de l'embase dudit connecteur sont répartis de façon à occuper plus de 1% de la surface totale du dispositif et notamment, plus de 5% de cette surface.

## Patentansprüche

1. Elektrischer Steckverbinder, insbesondere für eine medizinische Vorrichtung, die dazu bestimmt ist, auf der Haut eines Benutzers befestigt zu sein, einen Sockel umfassend, der dazu bestimmt ist, fest mit der Vorrichtung verbunden zu sein, und einen Kontakt, der dazu bestimmt ist, fest mit einem elektrischen Leiter verbunden zu sein, **dadurch gekennzeichnet, dass** der Kontakt (100, 110, 120, 400, 410, 420, 700, 2200, 2500, 2800) ein einziges Verbindungsmittel (102, 402, 702a, 2203, 2503, 2803) umfasst, und der Sockel (20, 50, 80, 2300, 2600, 2900) eine Vielzahl von Verbindungsmitteln umfasst (23, 53, 83, 2301, 2601, 2901), wobei jedes davon angepasst ist, um mit dem Verbindungsmittel des Kontakts zusammenzuwirken, um die Verbindung zwischen dem Sockel und dem Kontakt sicherzustellen.

2. Steckverbinder nach Anspruch 1, **dadurch gekennzeichnet, dass** der Abstand von Mitte zu Mitte zwischen zwei benachbarten Verbindungsmitteln des Sockels zwischen dem 1,5 und 10-fachendes Querschnitts dieser Verbindungsmittel umfasst.

3. Steckverbinder nach Anspruch 1 oder 2, **dadurch gekennzeichnet, dass** die Verbindungsmittel des Sockels auf gleichmäßige Weise auf der Oberfläche des Sockels verteilt sind.

4. Steckverbinder nach einem der Ansprüche 1 bis 3, **dadurch gekennzeichnet, dass** er magnetische Mittel sowohl auf dem Kontakt als auf dem Sockel umfasst.

5. Steckverbinder nach einem der Ansprüche 1 bis 4, **dadurch gekennzeichnet, dass** der Sockel (20, 50, 80) eine Vielzahl von Löchern (23, 53, 83) umfasst, und der Kontakt (100, 110, 120, 400, 410, 420, 700) einen Vorsprung (102, 402, 413, 423, 702a) umfasst.

6. Steckverbinder nach Anspruch 5, **dadurch gekennzeichnet, dass** der Sockel (20, 50, 80) aus einer Stapelung aus mindestens zwei Schichten gebildet ist, wobei eine von ihnen mindestens eine mechanische Tragfunktion erfüllt und die mindestens zwei Schichten auch eine elektrische Funktion und eventuell eine magnetische Funktion erfüllen, wobei die Löcher (23, 53, 83) des Sockels mindestens die Schicht, die die mechanische Tragfunktion erfüllt, durchqueren, und der Kontakt (100, 110, 120, 400, 410, 420, 700) eine Basis und einen Vorsprung (102, 402, 413, 423, 702a) umfasst, die eine elektrische Funktion und eventuell eine magnetische Funktion erfüllen.

7. Steckverbinder nach Anspruch 6, **dadurch gekennzeichnet, dass** die Stapelung, die den Sockel (20) bildet, eine Schicht (22) umfasst, die eine mechanische Tragfunktion erfüllt, und eine Schicht (21), die eine elektrische Funktion und eine eventuelle magnetische Funktion erfüllt, wobei die Löcher (23) die Stapelung durchqueren, wobei die elektrische Funktion und die eventuelle magnetische Funktion des Kontakts von der Basis (101, 110, 120) erfüllt wird (werden).

8. Steckverbinder nach Anspruch 6, **dadurch gekennzeichnet, dass** die Stapelung, die den Sockel (50) bildet, eine Schicht (52) umfasst, die eine elektrische Funktion erfüllt, und eine Schicht (51), die eine mechanische Funktion erfüllt, wobei die Schicht (52), die eine elektrische Funktion erfüllt, auch eine magnetische Funktion erfüllen kann, wobei die Löcher (53) nur die Schicht (51), die eine mechanische Funktion erfüllt, durchqueren, wobei die elektrische und eventuell magnetische Funktion des Kontakts (400, 410, 420) von dem Vorsprung (402, 413, 423) erfüllt wird.

9. Steckverbinder nach Anspruch 6, **dadurch gekennzeichnet, dass** die Stapelung, die den Sockel (80) bildet, sukzessive eine Schicht (82) umfasst, die eine elektrische Funktion erfüllt, eine Schicht (84), die eine mechanische Funktion erfüllt, und eventuell eine Schicht (81), die eine magnetische Funktion erfüllt, wobei die Löcher (83) die Schicht, die eine mechanische Funktion erfüllt, durchqueren, und, wenn sie vorhanden ist, die Schicht, die eine magnetische Funktion erfüllt, wobei die eventuelle magnetische Funktion des Kontakts (700) von der Basis (701) erfüllt wird, und die elektrische Funktion des Kontakts (700) durch den Vorsprung (702a) erfüllt wird.

10. Steckverbinder nach Anspruch 9, **dadurch gekennzeichnet, dass** die Schicht (84), die eine mechanische Funktion erfüllt, auch eine magnetische Funktion erfüllt.

11. Steckverbinder nach einem der Ansprüche 5 bis 10, **dadurch gekennzeichnet, dass** der Kontakt (1000) ein ringförmiges Teil (1001) umfasst, das auf der Basis (101) vorragt und sich um den Vorsprung (102) erstreckt, wobei der Sockel um jedes Loch eine ringförmige Öffnung (1101) umfasst, um das vorragende ringförmige Teil des Kontakts aufzunehmen.

12. Steckverbinder nach einem der Ansprüche 5 bis 11, **dadurch gekennzeichnet, dass** der Kontakt (1300) einen Vorsprung (1301) umfasst, der eine aufgeweitete Form aufweist, die sich verjüngt, während sie sich von der Basis (101) entfernt, wobei die Löcher des Sockels eine entsprechende Form umfassen, um diesen Vorsprung aufzunehmen.

13. Steckverbinder nach einem der Ansprüche 5 bis 11, **dadurch gekennzeichnet, dass** der Vorsprung (102) des Kontakts (1800) eine aufgeweitete Form aufweist, die sich erweitert, während sie sich von der Basis (101) entfernt, wobei die Löcher des Sockels eine entsprechende Form aufweisen, um diesen Vorsprung aufzunehmen.

14. Steckverbinder nach einem der Ansprüche 8 bis 13, **dadurch gekennzeichnet, dass** der Vorsprung (1504) des Kontakts (1500) zwei Teile (1501, 1503) umfasst, die eine elektrische Funktion und eventuell eine magnetische Funktion erfüllen, die durch ein Isoliermittel (1502) getrennt sind, wobei die Schicht des Sockels, die eine elektrische Funktion erfüllt, konzipiert ist, um zwei elektrische Signale zu übertragen.

15. Steckverbinder nach einem der Ansprüche 8 bis 13, **dadurch gekennzeichnet, dass** die Schicht des Sockels, die nicht von den Löchern durchquert wird, eine diskontinuierliche Schicht ist, die insbesondere in der Form eines Gitters (1401) oder unabhängiger und elektrisch nicht verbundener Teile (1912A, 1912B, 1916) vorliegt.

16. Steckverbinder nach einem der Ansprüche 7, 9 bis 15, **dadurch gekennzeichnet, dass** eine Schicht (1921), die von den Löchern durchquert wird, eine diskontinuierliche Schicht ist.

17. Steckverbinder nach einem der Ansprüche 5 bis 16, **dadurch gekennzeichnet, dass** der Vorsprung des Kontakts eine elastische Funktion erfüllt.

18. Steckverbinder nach einem der Ansprüche 6 bis 17, **dadurch gekennzeichnet, dass** die Schicht (21, 52, 82), die eine elektrische Funktion erfüllt, aus zwei getrennten und elektrisch nicht verbundenen Teilen (1912A, 1912B) besteht, wobei der Vorsprung (402, 413, 423; 702a) oder die Basis (100, 110, 120) des Kontakts es erlauben, diese zwei Teile elektrisch zu verbinden, wenn der Kontakt mit dem Sockel verbunden ist.

19. Steckverbinder nach einem der Ansprüche 1 bis 4, **dadurch gekennzeichnet, dass** der Sockel (2300, 2600, 2900) eine Vielzahl von Vorsprüngen (2301, 2601, 2901) umfasst und der Kontakt (2200, 2500, 2800) einen Hohlraum (2203, 2503, 2803) umfasst.

20. Steckverbinder nach Anspruch 19, **dadurch gekennzeichnet, dass** der Sockel (2300, 2600, 2900) mindestens eine Schicht umfasst, die eine mechanische Tragfunktion erfüllt, und die Vielzahl von Vorsprüngen direkt auf der mechanischen Tragschicht oder auf einer Zwischenschicht hergestellt ist, wobei die Vorsprünge, die Schicht eine mechanische Tragfunktion erfüllen oder die Zwischenschicht eine elektrische Funktion und eventuell eine mechanische Funktion erfüllt, und der Kontakt (2200, 2500, 2800) ein zylindrisches Teil umfasst, das den Hohlraum definiert, der durch eine Basis verschlossen ist, wobei die elektrische Funktion des Kontakts von dem zylindrischen Teil oder der Basis erfüllt wird und die eventuelle magnetische Funktion des Kontakts von dem zylindrischen Teil oder der Basis erfüllt wird.

21. Steckverbinder nach Anspruch 20, **dadurch gekennzeichnet, dass** der Sockel (2300) auf der Schicht (2303), die eine mechanische Tragfunktion erfüllt, eine eventuelle Zwischenschicht (2302) umfasst, wobei die elektrische Funktion und die eventuelle magnetische Funktion von der Schicht (2303) oder der Zwischenschicht (2302) erfüllt wird (werden), wobei die Vorsprünge (2301) auf der Schicht (2303) oder auf der Zwischenschicht (2302) hergestellt sind, wobei die elektrische Funktion und die eventuelle magnetische Funktion des Kontakts (2200) von dem zylindrischen Teil (2201) erfüllt wird (werden).

22. Steckverbinder nach Anspruch 20, **dadurch gekennzeichnet, dass** der Sockel (2600) auf der Schicht (2602), die eine mechanische Tragfunktion erfüllt, Vorsprünge (2601) umfasst, die direkt auf dieser mechanischen Tragschicht hergestellt sind, wobei diese Vorsprünge eine elektrische Funktion und eventuell eine magnetische Funktion erfüllen, wobei die elektrische Funktion und die eventuelle magnetische Funktion des Kontakts (2500) von der Basis (2502) erfüllt wird (werden).

23. Steckverbinder nach Anspruch 20, **dadurch gekennzeichnet, dass** der Sockel (2900) auf der Schicht, die eine mechanische Tragfunktion erfüllt, eine Zwischenschicht (2902) umfasst, die eine elektrische Funktion erfüllt, wobei die Vorsprünge (2901) auf dieser Zwischenschicht hergestellt sind und eventuell eine magnetische Funktion erfüllen, wobei das zylindrische Teil (2801) des Kontakts eine elektrische Funktion erfüllt und seine Basis (2802) eventuell eine magnetische Funktion erfüllt.

24. Steckverbinder nach Anspruch 23, **dadurch gekennzeichnet, dass** die Vorsprünge des Sockels eine elektrische Funktion erfüllen und die Zwischenschicht eventuell eine magnetische Funktion erfüllt.

25. Steckverbinder nach einem der Ansprüche 1 bis 24, **dadurch gekennzeichnet, dass** er eine Vielzahl von Kontakten umfasst, deren Anzahl streng kleiner ist als die der Verbindungsmittel.

26. Medizinische Vorrichtung, die dazu bestimmt ist, auf der Haut eines Benutzers befestigt zu sein, einen elektrischen Steckverbinder nach einem der Ansprüche 1 bis 25 umfassend.

27. Vorrichtung nach Anspruch 26, wobei die Verbindungsmittel des Sockels des Steckverbinders derart verteilt sind, dass sie mehr als 1 % der Gesamtoberfläche der Vorrichtung und insbesondere mehr als 5 % dieser Oberfläche belegen.

## Claims

1. An electrical connector, in particular for a medical device intended to be fixed on the skin of a user, comprising a base secured to the device and a plug intended to be secured to an electrical conductor, **characterized in that** the plug (100, 100, 120, 400, 410, 420, 700, 2200, 2500, 2800) comprises a single connection means (102, 402, 702a, 2203, 2503, 2803) and the base (20, 50, 80, 2300, 2600, 2900) comprises a plurality of connection means (23, 53, 83, 2301, 2601, 2901), each of them being suitable for cooperating with the connecting means of the plug to ensure the connection between the base and the plug.

2. The connector according to claim 1, **characterized in that** the center-to-center distance between two adjacent connecting means of the base is comprised between 1.5 and 10 times the section of these connecting means.

3. The connector according to claim 1 or 2, **characterized in that** the connecting means of the base are preferably distributed uniformly over the surface of the base.

4. The connector according to one of claims 1 to 3, **characterized in that** it includes magnetic means on both the plug and the base.

5. The connector according to one of claims 1 to 4, **characterized in that** the base (20, 50, 80) includes a plurality of holes (23, 53, 83) and the plug (100, 110, 120, 400, 410, 420, 700) includes a protuberance (102, 402, 413, 423, 702a).

6. The connector according to claim 5, **characterized in that** the base (20, 50, 80) is made up of a stack of at least two layers, one of them performing at least a mechanical support function and said at least two layers also performing an electrical function and optionally a magnetic function, the holes (23, 53, 83) of the base traversing at least the layer acting as mechanical support, and the plug (100, 110, 120, 400, 410, 420, 700) comprising a base and a protuberance (102, 402, 413, 423, 702a) performing an electrical function and optionally a magnetic function.

7. The connector according to claim 6, **characterized in that** the stack making up the base (20) comprises a layer (22) performing a mechanical support function and a layer (21) performing an electrical function and optionally a magnetic function, the holes (23) traversing the stack, the electrical function and optionally magnetic function of the plug is performed by the base (101, 110, 120).

8. The connector according to claim 6, **characterized in that** the stack making up the base (50) comprises a layer (52) performing an electrical function and a layer (51) performing a mechanical function, the layer (52) performing an electrical function also being able to perform a magnetic function, the holes (53) only traversing the layer (51) performing a mechanical function, the electrical function and optionally magnetic function of the plug (400, 410, 420) being performed by the protuberance (402, 413, 423).

9. The connector according to claim 6, **characterized in that** the stack making up the base (80) successively comprises a layer (82) performing an electrical function, a layer (84) performing a mechanical function and optionally a layer (81) performing a magnetic function, the holes (83) traversing the layer performing a magnetic function and, when it is present, the layer performing a magnetic function, any magnetic function of the plug (700) being performed by the base (701) and the electrical function of the plug (700) being performed by the protuberance (702a).

10. The connector according to claim 9, **characterized in that** the layer (84) performing a mechanical function also performs a magnetic function.

11. The connector according to one of claims 5 to 10, **characterized in that** the plug (1000) comprises an annular part (1001) protruding on the base (101) and extending around the protuberance (102), the base comprising, around each hole, an annular opening (1101) to receive the protruding annular part of the plug.

12. The connector according to one of claims 5 to 11, **characterized in that** the plug (1300) includes a protuberance (1301) having a flared shape becoming narrower as it moves away from the base (101), the holes of the base including a corresponding shape to receive this protuberance.

13. The connector according to one of claims 5 to 11, **characterized in that** the protuberance (102) of the plug (1800) has a flared shape becoming wider as it moves away from the base (101), the holes of the base having a corresponding shape to receive this protuberance.

14. The connector according to one of claims 8 to 13, **characterized in that** the protuberance (1504) of the plug (1500) comprises two parts (1501, 1503) performing an electrical function and optionally a magnetic function, separated by an insulator (1502), the layer of the base that performs an electrical function being designed to transmit to electrical signals.

15. The connector according to one of claims 8 to 13, **characterized in that** the layer of the base that is not traversed by the holes is a discontinuous layer, in particular assuming the form of a grid (1401) or independent parts (1912A, 1912B, 1916) that are not electrically connected.

16. The connector according to one of claims 7, 9 to 15, **characterized in that** a layer (1921) traversed by the holes is a discontinuous layer.

17. The connector according to one of claims 5 to 16, **characterized in that** the protuberance of the plug performs an elastic function.

18. The connector according to one of claims 6 to 17, **characterized in that** the layer (21, 52, 82) performing an electrical function is made up of two separate parts (1912A, 1912B) that are not electrically connected, the protuberance (402, 413, 423; 702a) or the base (100, 110, 120) of the plug making it possible to electrically connect these two parts when the plug is connected to the base.

19. The connector according to one of claims 1 to 4, **characterized in that** the base (2300, 2600, 2900) includes a plurality of protuberances (2301, 2601, 2901) and the plug (220, 2500, 2800) includes a cavity (2203, 2503, 2803).

20. The connector according to claim 19, **characterized in that** the base (2300, 2600, 2900) comprises at least one layer performing a mechanical support function and the plurality of protuberances is made directly on the mechanical support layer or on an intermediate layer, the protuberances, the layer performing a mechanical support function or the intermediate layer performing an electrical function and optionally a magnetic function, and the plug (2200, 2500, 2800) comprises a cylindrical part defining the cavity that is closed by a base, the electrical function of the plug being performed by the cylindrical part or the base and the potential magnetic function of the plug being performed by the cylindrical part or the base.

21. The connector according to claim 20, **characterized in that** the base (2300) comprises, on the layer (2303) performing a mechanical support function, a potential intermediate layer (2302), the electrical function and the potential magnetic function being performed by the layer (2303) or the intermediate layer (2302), the protuberances (2301) being made on the layer (2303) or on the intermediate layer (2302), the electrical function and the potential magnetic function of the plug (2200) being performed by the cylindrical part (2201).

22. The connector according to claim 20, **characterized in that** the base (2600) comprises, on the layer (2602) performing a mechanical support function, protuberances (2601) made directly on this mechanical support layer, these protuberances performing an electrical function and optionally a magnetic function, the electrical function and the potential magnetic function of the plug (2500) being performed by the base (2502).

23. The connector according to claim 20, **characterized in that** the base (2900) comprises, on the layer performing a mechanical support function, an intermediate layer (2902) performing an electrical function, the protuberances (2901) being made on this intermediate layer and optionally performing a magnetic function, the cylindrical part (2801) of the plug performing an electrical function and its base (2802) optionally performing a magnetic function.

24. The connector according to claim 23, **characterized in that** the protuberances that perform an electrical function and the intermediate layer that optionally performs a magnetic function.

25. The connector according to one of claims 1 to 24, **characterized in that** it includes a plurality of plugs, the number of which is strictly smaller than that of the connecting means of the base.

26. A medical device intended to be fixed on the skin of a user, comprising an electrical connector according to one of claims 1 to 25.

27. The device according to claim 26, wherein the connecting means of the base are advantageously distributed so as to occupy more than 1 % of the total surface area of the device, and in particular more than 5% of this surface area.
